# EUROPEAN PATENT APPLICATION

(11) **EP 4 578 511 A1**
(43) Date of publication of application: **02.07.2025**
(21) Application number: 23857087.3
(22) Date of filing: 26.07.2023
(51) Int. Cl.: A61Q 1/00, A61Q 19/00, A61K 8/37, A61K 8/891

(54) **LIQUID OILY COMPOSITION, AND COSMETIC MATERIAL CONTAINING LIQUID OILY COMPOSITION**

(30) Priority: 23.08.2022 JP 2022132567
(71) Applicant: THE NISSHIN OILLIO GROUP, LTD., Tokyo 104-8285 (JP)
(72) Inventor: INOKOISHI Yogo, Yokohama-shi, Kanagawa 235-8558 (JP); KACHI Hisanori, Yokohama-shi, Kanagawa 235-8558 (JP); OYAMA Keiichi, Yokohama-shi, Kanagawa 235-8558 (JP)
(74) Representative: Hasegawa, Kan
(86) International application number: PCT/JP2023/027326
(87) International publication number: WO 2024/042971

(57) **Abstract**

A liquid oily composition according to the present invention contains: component (A) of dimethicone having a kinematic viscosity of 4-12 mm²/s at 25°C; component (B) of an ester oil that is liquid at 25°C and has a dimethicone compatibility value of at least 1.75 as calculated by formula (1); and component (C) of an ester oil that is liquid at 25°C and has a dimethicone compatibility value of at least 1.75 as calculated by the following formula (I), wherein the component (A) is separated from a mixture that is uniform and transparent at 25°C and is prepared by adding 8 parts by mass of macadamia nut oil with respect to 100 parts by mass of the liquid oil composition.

## Description

### TECHNICAL FIELD

The present invention relates to a liquid oily composition, and a cosmetic material containing the liquid oily composition.

Priority is claimed on Japanese Patent Application No. 2022-132567, filed August 23, 2022, the content of which is incorporated herein by reference.

### BACKGROUND ART

The causes of smearing of cosmetic materials typically include internal factors and external factors.

Examples of the internal factors include contact with the cosmetic material of human secretions including moisture such as sweat and tears, and oily components such as sebum. The external factors include adhesion of the cosmetic material to masks or clothing or the like as a result of contact with the mask or clothing. In the following description, this type of adhesion of the cosmetic material is referred to as "secondary adhesion". Protecting cosmetic films from the types of factors described above, thereby ensuring good persistence of the cosmetic film is a significant issue.

A method for addressing cosmetic smearing caused by sweat by adding a water-repellent substrate such as a silicone resin to the cosmetic material has already been proposed. Further, a method for addressing cosmetic smearing caused by sebum by adding a water-repellent, oil-repellent substrate such as a fluorine-based resin to the cosmetic material has also been proposed.

For example, Patent Document 1 describes a cosmetic material containing a fluorine-modified silicone obtained by introducing fluoroalkyl groups into dimethyl silicone. Further, Patent Document 2 describes an emulsion cosmetic in which, by adding a specific powder in addition to the silicone resin of a film-forming agent, effects are obtained that include favorable spreading, smoothness, low impact on the skin, good persistence of the cosmetic effect over long periods, and excellent uniformity of the cosmetic film.

However, although fluorine-modified silicones obtained by introducing fluoroalkyl groups into dimethyl silicone and silicone resins that act as film-forming agents exhibit good persistence of the cosmetic film, they tend to have poor compatibility with other components. As a result, stability problems may occur such as separation of the cosmetic material during storage, and there are sometimes limits on the sensations that can be achieved due to restrictions on the other blend components used in order to achieve satisfactory compatibility.

Furthermore, in order to form a cosmetic film using a silicone resin that acts as film-forming agent, a period of about 15 minutes is required following application to the skin to allow evaporation of the solvent, and a feeling of tightness is also sometimes noticeable following formation of the cosmetic film.

In light of this type of background, there is a demand for the development of a cosmetic raw material that suppresses secondary adhesion of the cosmetic material, has water repellency with strength and persistence, exhibits favorable compatibility with the other cosmetic material components, and has few formulation restrictions.

### Citation List

### Patent Document

Patent Document 1: Japanese Unexamined Patent Application, First Publication No. Hei-06-184312
Patent Document 2: Japanese Unexamined Patent Application, First Publication No. 2006-265213

### SUMMARY OF INVENTION

### Technical Problem

The present invention has been developed in light of the above circumstances, and has the objects of providing a liquid oily composition which, when applied to the skin, has an inhibitory effect on secondary adhesion and imparts water repellency with strength and persistence, and also providing a cosmetic material containing the liquid oily composition.

### Solution to Problem

The inventors of the present invention found that in uniform liquid oily compositions containing specific proportions of dimethicone and at least two types of ester oils having differing compatibility with the dimethicone, the objects described above could be achieved by using suitable ester oils selected on the basis of their dimethicone compatibility value, calculated from the molecular structural features of the ester oil. **In** other words, they found that a cosmetic material containing this liquid oily composition, upon mixing with sebum, was able to impart an excellent inhibitory effect on secondary adhesion, and excellent water repellency with strength and persistence, thus enabling them to complete the present invention.

In other words, the present invention includes the following aspects.
[1] A liquid oily composition containing:
   a component (A): a dimethicone having a kinematic viscosity at 25°C within a range from 2 to 12 mm²/s,
   a component (B): an ester oil that is liquid at 25°C and has a dimethicone compatibility value, determined using formula (I) shown below, of at least 1.75, and
   a component (C): an ester oil that is liquid at 25°C and has a dimethicone compatibility value, determined using formula (I) shown below, of less than 1.75, wherein
   the liquid oily composition is transparent and uniform at 25°C, and
   the component (A) is separated from a mixture prepared by adding 8 parts by mass of macadamia nut oil to 100 parts by mass of the liquid oily composition.
      [Numerical Formula 1] Dimethicone compatibility value of the ester oil = 10,000 × (carbon branching value + oxygen value) / (molecular weight of the ester oil)2 (I) Carbon branching value = (the number of carbon branches within the molecular structure of the ester oil × 12 Oxygen value = (-3 × the number of hydroxyl groups within the molecular structure of the ester oil × 16) + (0.5 × the number of ester groups within the molecular structure of the ester oil × 2 × 16) + (-0.1 × the number of ether groups in the ester oil × 16)
[2] The liquid oily composition according to [1], wherein
   a mass ratio ((B)/(C)) of the component (C) to the component (B) is within a range from 0.32 to 18.00, and
   a mass ratio ({(B)+(C)}/(A)) of the component (A) to the total of the component (B) and the component (C) is within a range from 1.50 to 12.33.
[3] The liquid oily composition according to [1] or [2], wherein
   the component (B) is one type, or at least two types, of ester oil selected from the group consisting of isononyl isononanoate, isotridecyl isononanoate, neopentyl glycol diethylhexanoate, glyceryl triethylhexanoate, glyceryl triisostearate (having a 2-(1,3,3-trimethylbutyl)-5,7,7-trimethyloctyl structure in the aliphatic chain), diisostearyl malate (having a 2-(1,3,3-trimethylbutyl)-5,7,7-trimethyloctyl structure in the aliphatic chain), pentaerythrityl tetraethylhexanoate, neopentyl glycol dicaprate, propanediol di(caprylate/caprate), polyglyceryl-2 triisostearate (having a 2-(1,3,3-trimethylbutyl)-5,7,7-trimethyloctyl structure in the aliphatic chain), cetyl ethylhexanoate, ethylhexyl palmitate, glyceryl tri(caprylate/caprate), and dipentaerythrityl pentaisostearate (having a 2-(1,3,3-trimethylbutyl)-5,7,7-trimethyloctyl structure in the aliphatic chain).
[4] The liquid oily composition according to any one of [1] to [3], wherein
   the component (C) is one type, or at least two types, of ester oil selected from the group consisting of propanediol diisostearate, trimethylolpropane triisostearate, pentaerythrityl tetraisostearate, polyglyceryl-2 tetraisostearate, polyglyceryl-2 triisostearate, and ethylhexyl hydroxystearate.
[5] The liquid oily composition according to any one of [1] to [4], wherein
   the viscosity at 25°C of a mixture of the component (B) and the component (C) is within a range from 30 to 1,200 mPa·s.
[6] A cosmetic material containing the liquid oily composition according to any one of [1] to [5].
[7] The cosmetic material according to [6], further containing a component (D): a powder.
[8] The cosmetic material according to [6] or [7], further containing a component (E): water, and a component (F): a surfactant, wherein
   the cosmetic material is an oil-in-water emulsion cosmetic material or a water-in-oil emulsion cosmetic material.
[9] A method for producing a cosmetic material, including blending the liquid oily composition of any one of [1] to [5] as a raw material.

### Advantageous Effects of Invention

The present invention is able to provide a liquid oily composition which produces an inhibitory effect on secondary adhesion of cosmetic materials, and imparts water repellency with strength and persistence, as well as providing a cosmetic material containing the liquid oily composition.

### BRIEF DESCRIPTION OF THE DRAWINGS

[FIG. 1] Captured photographs (actual images) of Example 1 and Comparative Example 1, as well as images obtained by subjecting those photographs to binarization processing are indicated.
[FIG. 2] Captured photographs (actual images) of Example 42 and Comparative Example 50, as well as images obtained by subjecting those photographs to binarization processing are indicated.

### DESCRIPTION OF EMBODIMENTS

When describing numerical ranges, a range that is "at least Abut not more than B" may sometimes be described using the expression "from A to B". For example, an expression "from 1 to 10 parts by mass" means a numerical range from 1 part by mass to 10 parts by mass, including the lower limit value (1 part by mass) and the upper limit value (10 parts by mass), in other words, "at least 1 part by mass but not more than 10 parts by mass".

### <Liquid Oily Composition>

First is a description of an overview of the liquid oily composition of the present embodiment.

The liquid oily composition of the present embodiment contains a specific dimethicone described below (component (A)) and specific ester oils (component (B) and component (C)). The component (A), the component (B) and the component (C) are described below in detail.

Component (A): one type, or at least two types, of dimethicone having a kinematic viscosity at 25°C within a range from 2 to 12 mm²/s.

Component (B): one type, or at least two types, of ester oil that are liquid at 25°C and have a dimethicone compatibility value, determined using formula (I) shown below, of at least 1.75.

Component (C): one type, or at least two types, of ester oil that are liquid at 25°C and have a dimethicone compatibility, determined using formula (I) shown below, of less than 1.75.

The liquid oily composition of the present embodiment has the following two properties.

The liquid oily composition is transparent and uniform at 25°C (hereinafter referred to as "compatibility 1").

If 8 parts by mass of macadamia nut oil is added to 100 parts by mass of the liquid oily composition, the added macadamia nut oil is not compatible (hereinafter referred to as "compatibility 2").

In those cases where the external appearance of the liquid oily composition exhibits a transparent and uniform state, the components that constitute the liquid oily composition are defined as having mutual compatibility, or being compatible. In this description, the expression "compatible" or "has compatibility" means that when the mixture (the liquid oily composition) is inspected visually, the mixture has a transparent state with no separation, cloudiness or turbidity, whereas the expression "not compatible" or "lacks compatibility" means that when the mixture is inspected visually, separation, cloudiness or turbidity is observed.

### (Compatibility 1)

In a liquid oily composition that satisfies compatibility 1 described above, the various components that constitute the liquid oily composition are mutually compatible at 25°C.

### (Compatibility 2)

The expression "is not compatible" relating to compatibility 2 described above means that when a mixture obtained by adding macadamia nut oil to the liquid oily composition is inspected visually, separation, cloudiness or turbidity is observed.

Macadamia nut oil and the component (A) are not compatible regardless of the ratio at which they are mixed, whereas both the combination of macadamia nut oil and the component (B), and the combination of macadamia nut oil and the component (C) are compatible regardless of the ratio at which they are mixed. As a result of such a difference in compatibility of each component with macadamia nut oil, when macadamia nut oil is mixed with the liquid oily composition, the macadamia nut oil is compatible with the component (B) and the component (C), but the component (A) is separates therefrom. In other words, the liquid oily composition of the present embodiment, which satisfies both compatibility 1 and compatibility 2, is a transparent and uniform liquid composition prior to mixing with macadamia nut oil, but loses compatibility upon mixing with macadamia nut oil.

In the present embodiment, the macadamia nut oil is used as a substitute of sebum. The constituent fatty acids that constitute macadamia nut oil are similar to the constituent fatty acids of the triglycerides that constitute sebum. Consequently, macadamia nut oil and sebum exhibit similar behaviors in terms of compatibility with the dimethicone of the component (A), the ester oil of the component (B) and the ester oil of the component (C). Accordingly, in the present embodiment, the macadamia nut oil is used instead of sebum to confirm the behavior of the liquid oily composition when sebum mixes with the liquid oily composition. More specifically, the fact that the liquid oily composition lacks compatibility with sebum when applied to the skin is confirmed by investigating the compatibility upon addition of macadamia nut oil (compatibility 2).

Based on the relative relationship between the amount of sebum on the skin of an adult and the amount of cosmetic material typically applied, when the liquid oily composition is applied to the skin, the amount of sebum mixed with the liquid oily composition is about 8 parts by mass per 100 parts by mass of the liquid oily composition. In compatibility 2, in order to confirm the compatibility under a state as similar as possible to that when the liquid oily composition mixes with sebum on the skin, the "compatibility when 8 parts by mass of macadamia nut oil are added per 100 parts by mass of the liquid oily composition" is used as the criteria for ascertaining compatibility.

In other words, the liquid oily composition of the present embodiment which exhibits both compatibility 1 and compatibility 2 is a transparent and uniform liquid composition prior to application to the skin, but when applied to the skin, contact and mixing of the liquid oily composition with sebum on the skin causes the liquid oily composition to lose compatibility, thereby separating the component (A) from the component (B) and the component (C). The separated component (A) forms a film of the component (A) on the skin (hereinafter, this film of the component (A) is sometimes referred to as a "silicone film"). This silicone film formed following separation from the liquid oily composition exhibits water repellency, and is a silicone film having favorable flexibility that produces no feeling of tightness in comparison with cosmetic films formed using conventional technology. Moreover, this silicone film serves as a protective film, thereby achieving an inhibitory effect on secondary adhesion.

Typical film-forming agents are compose of a solid and a solvent that dissolves that solid. By applying this film-forming agent to the skin and then evaporating the solvent, the solid is hardened and retained on the skin to form a film. It is thought that because this film is solid, the shape of the film cannot be freely altered, and the film is unable to adapt to movement of the skin, resulting in a feeling of tightness. In contrast, the silicone film formed in the present embodiment is liquid rather than solid, and it is surmised that because the film is not hardened on the skin, a feeling of tightness is not generated.

As described above, a liquid oily composition that satisfies compatibility 1 (transparent and uniform at 25°C) and compatibility 2 (is not compatible in the presence of 8% by mass of macadamia nut oil) mixes with sebum when applied to the skin, thereby forming a silicone film (a film of the component (A)) on the skin, and the silicone film suppresses secondary adhesion and exhibits water repellency. Further, the formed silicone film exhibits flexibility and produces no feeling of tightness.

In other words, the liquid oily composition applied to the skin forms a coating film having a layer structure in which a thin film composed of the residual liquid oily composition from which the component (A) has been removed as a result of addition of sebum to the liquid oily composition, and a silicone film formed on the surface of the thin film are formed in this order from the skin side. Hereinafter, the thin film composed of the residual liquid oily composition from which the component (A) has been removed as a result of addition of sebum to the liquid oily composition is sometimes referred to as the "ester oil film".

The effects of the present embodiment, namely excellent inhibition on secondary adhesion and excellent water repellency with strength and persistence, are achieved by the silicone film formed on the surface of the coating film of the liquid oily composition. By applying the liquid oily composition that satisfies compatibility 2 to the skin, a silicone film is formed on the surface of the thin film (the ester oil film). Accordingly, even if the various effects of the liquid oily composition such as inhibition on secondary adhesion, flexibility of the cosmetic film, strength of the water repellency, and persistence of the water repellency are not specifically confirmed by testing, provided the liquid oily composition according to the present invention satisfies the condition of compatibility 2, it may be stated that the composition is able to produce those effects.

### <<Component (A)>>

Next is a description of the component (A) used in the liquid oily composition according to the present invention.

Silicone oil is a generic term for liquid substances having a structure of general formula (1) shown below. The component (A) used in the liquid oily composition of the present embodiment is, as indicated by formula (2) shown below, a substance in which the R portion structures are methyl groups, and is typically referred to as a dimethicone.
[Chemical formula 1]

H-(O-Si(R)₂-O-Si(R)₂)ₙ-O-H R=CH₃ or Phenyl (1)

[Chemical formula 2]

H-(O-Si(CH₃)₂-O-Si(CH₃)₂)ₙ-O-H (2)

In dimethicones, the kinematic viscosity increases as the degree of polymerization increases. Different types of dimethicones are typically distinguished using the respective values of the kinematic viscosity.

In the present invention and the present description, the kinematic viscosity is the value obtained by dividing the viscosity at 25°C by the density of the fluid viscous liquid at the same temperature. The unit of the kinematic viscosity is typically expressed in square millimeters per second (mm²/s).

The viscosity of a dimethicone can be measured using the method disclosed in "55. Viscosity Measurement Methods, Method 2, Rotary Viscometer Method" of the 2021 Japanese Standards of Quasi-drug Ingredients (Japan Ministry of Health, Labour and Welfare, Pharmaceutical Safety and Environmental Health Bureau, Pharmaceutical Evaluation Division). Specifically, the viscosity can be measured using a B-type viscometer, by selecting the rotor and rotational rate appropriately to achieve a reading within a range from 10 to 90, and then reading the value displayed 60 seconds after the start of rotation.

The density of a dimethicone can be measured using a specific gravity bottle.

The dimethicone of the component (A) used in the liquid oily composition of the present embodiment has a kinematic viscosity at 25°C that is typically within a range from 4 to 12 mm²/s, preferably from 5 to 12 mm²/s, and more preferably from 5 to 10 mm²/s. By ensuring that the kinematic viscosity at 25°C of the dimethicone of the component (A) falls within this range, a satisfactory inhibitory effect on secondary adhesion can be obtained. As the component (A), one type of the dimethicone having a kinematic viscosity within the above-mentioned range may be used, or a mixture of at least two types thereof may be used.

A commercially available product may be used as the dimethicone of the component (A). Examples of such commercially available products include a dimethicone having a kinematic viscosity at 25°C of 5 mm²/s (product name: KF-96L-5cs, manufactured by Shin-Etsu Chemical Co., Ltd.) and a dimethicone having a kinematic viscosity at 25°C of 10 mm²/s (product name: KF-96A-10cs, manufactured by Shin-Etsu Chemical Co., Ltd.).

### <<Component (B) and Component (C)>>

Next are descriptions of the component (B) and the component (C) used in the liquid oily composition according to the present invention.

The component (B) is an ester oil that is liquid at 25°C and has a dimethicone compatibility value of at least 1.75. The component (C) is an ester oil that is liquid at 25°C and has a dimethicone compatibility value of less than 1.75.

### [Dimethicone Compatibility Value of Ester Oils]

The dimethicone compatibility value of the ester oil can be determined from formula (I) shown below.
[Numerical Formula 2] Dimethicone compatibility value of the ester oil = 10,000 × (carbon branching value + oxygen value) / (molecular weight of the ester oil)2 (I) Carbon branching value = (the number of carbon branches within the molecular structure of the ester oil × 12 Oxygen value = (-3 × the number of hydroxyl groups within the molecular structure of the ester oil × 16) + (0.5 × the number of ester groups within the molecular structure of the ester oil × 2 × 16) + (-0.1 × the number of ether groups in the ester oil × 16)

The "dimethicone compatibility value of the ester oil" is a value for evaluating the compatibility of the ester oil and dimethicone. The dimethicone compatibility value of the ester oil is calculated from the above formula (1), using a carbon branching value and an oxygen value determined on the basis of the molecular structure of the ester oil. A larger dimethicone compatibility value of the ester oil indicates better compatibility between the ester oil and dimethicone, whereas a smaller dimethicone compatibility value indicates inferior compatibility.

The carbon branching value is a value that reflects the number of terminal methyl groups within the ester oil which promote compatibility between the ester oil and dimethicone. The numerical value of 12 used in the calculation of the carbon branching value is the atomic weight of carbon.

Generally, substances having similar structures within their respective molecular structures are more likely to exhibit good compatibility. As indicated by formula (2) shown above, dimethicone is a silicone oil having methyl groups as side chains (a polydimethylsiloxane), and has many methyl groups within the molecular structure. Accordingly, the larger the number of methyl groups as side chains in the ester oil, the better the compatibility between the ester oil and dimethicone tends to become.

On the other hand, the oxygen value is a value that reflects the polar state around oxygen atoms within the ester oil which inhibits compatibility between the ester oil and dimethicone. The numerical value of 16 used in the calculation of the oxygen value is the atomic weight of oxygen.

A dimethicone is a silicone oil having methyl groups as side chains, and has low polarity. Accordingly, as the polarity of an ester oil increases, the ester oil and dimethicone become less compatibility. In view of such a background, coefficients, such as -3, 0.5 and -0.1, that reflect the degree of the contribution to the compatibility depending on each functional group are used in the formula for calculating the oxygen value.

In this description, the number of carbon branches used in calculating the carbon branching value is a number determined by the formula: "the number of quaternary carbons × 2 + the number of tertiary carbons", when the branched structure of an ester oil is known.

When the branched structure of an ester oil is not known, the number of carbon branches used in calculating the carbon branching value can be determined from a ¹H NMR chart. First, an ¹H NMR chart of a fatty acid and/or alcohol that constitute the ester oil is obtained. From the obtained ¹H NMR chart, the number of carbon branches in each fatty acid and alcohol is calculated. The number of carbon branches in the ester oil is then calculated from the determined numbers of carbon branches in the fatty acid and alcohol.

Specific examples of the method employed include the following method. There are no particular limitations on the apparatus used to measure the ¹H NMR, and any commercially available apparatus may be used.

The ester oil to be analyzed may be subjected to hydrolysis as a pretreatment before analyzing the fatty acid and alcohol that constitute the ester oil. First, the ester oil to be analyzed is saponified using potassium hydroxide. Next, the excess potassium hydroxide is neutralized with hydrochloric acid, and the generated alcohol is extracted with a solvent and collected. The saponified product of the fatty acid remaining following removal of the alcohol is neutralized once more with hydrochloric acid, and then the fatty acid is extracted with a solvent and collected. The solvent is removed from each of the collected products containing the alcohol and the fatty acid, and each of the residues is used as a ¹H NMR sample.

First, the fatty acid and/or alcohol that constitute the ester oil are subjected to ¹H NMR measurements. In the resulting chart, the area ratio of the peak attributable to protons derived from methyl groups is calculated when the area of the peak attributable to protons derived from either carboxylic acid groups of the fatty acid or hydroxyl groups of the alcohol is defined to be 1. Next, the area ratio of the peak attributable to protons derived from methyl groups is divided by 3, followed by subtracting 1, which corresponds to the number of terminal methyl group on the carbon main chain, therefrom. The number obtained by this method is the number of carbon branches included in the fatty acid or alcohol. Finally, the number of carbon branches of the ester oil can be determined by summing the number of carbon branches of the fatty acid and/or alcohol that constitute the ester oil in accordance with the blend molar ratio used to carry out the ester synthesis. This method for calculating the number of carbon branches from a ¹H NMR chart may also be used when the fatty acid and/or alcohol is a mixture, or the number of branches is not an integer.

In the case where CDCl₃ is used as a solvent and tetramethylsilane is used as a standard substance to carry out the analysis with ¹H NMR, protons derived from methyl groups cause a chemical shift within a range from 1.2 to 0.8 ppm, protons derived from hydroxyl groups cause a chemical shift within a range from 4.0 to 4.5 ppm, and protons derived from carboxylic acid groups cause a chemical shift within a range from 10 to 13 ppm.

### [Calculation of Formula (I)]

The formula (I) can be determined in the following manner, as disclosed in detail in the below-mentioned examples.

First, in order to calculate the dimethicone compatibility value of ester oils, the compatibility of each mixture of dimethicones having various kinematic viscosity values and various ester oils at a mass ratio of 1:1 is ascertained at 25°C.

Generally, it is known that as the viscosity of the dimethicone increases, the compatibility with ester oils tends to deteriorate. Accordingly, an ester oil that is compatible with a dimethicone of high viscosity will also be compatible with dimethicones of lower viscosity, and therefore has compatibility with dimethicones across a broad range of viscosity values, and can be called an ester oil of superior compatibility with dimethicone.

Next, the number of carbon branches of each used ester oil is determined from the ¹H NMR chart, and the number of hydroxyl groups, the number of ester groups and the number of ether groups are determined from the structures of the fatty acid and alcohol that constitute the ester oil, and the hydroxyl value of the ester oil. The carbon branching value is then calculated from the number of carbon branches, and the oxygen value is calculated from the number of hydroxyl groups, the number of ester groups and the number of ether groups.

The formula (I) is derived by matching the compatibility at 25°C of the mixtures of dimethicones of various kinematic viscosity values and various ester oils at a mass ratio of 1:1 with the structural features of the ester oils. The sum of the carbon branching value and the oxygen value is multiplied by 10,000 and then divided by the square of the molecular weight of the ester oil in accordance with formula (I), and the resulting value is deemed as the dimethicone compatibility value.

An ester oil with superior compatibility with dimethicone tends to exhibit a larger dimethicone compatibility value derived from the structural feature of the ester oil. On the other hand, an ester oil with inferior compatibility with dimethicone tends to exhibit a smaller dimethicone compatibility value derived from the structural feature of the ester oil.

For example, it can be concluded that an ester oil having a dimethicone compatibility value of at least 1.75 is compatible with dimethicone having a kinematic viscosity of 10 mm²/s based on the compatibility of mixtures of dimethicone having a kinematic viscosity of 10 mm²/s with various esters at a mass ratio of 1:1, and the dimethicone compatibility value calculated using the above formula (I).

In such a manner, ester oils having a dimethicone compatibility value calculated from formula (I) of at least 1.75 can be deemed to be usable as the component (B), even without confirming the compatibility with dimethicone by experimental testing. In a similar manner, ester oils having a dimethicone compatibility value calculated from formula (I) of less than 1.75 can be deemed to be usable as the component (C), even without confirming the compatibility with dimethicone by experimental testing.

The formula (I) of the present embodiment can also be used as an evaluation criterion of the compatibility of a mixture prepared by mixing at least two types of dimethicone having different kinematic viscosities, for example, dimethicones having kinematic viscosities of 5 mm²/s and 10 mm²/s, and an ester oil at a mass ratio of 1:1.

Any ester oil that is liquid at 25°C and has a dimethicone compatibility value of at least 1.75 may be used as the ester oil of the component (B). Further, any ester oil that is liquid at 25°C and has a dimethicone compatibility value of less than 1.75 may be used as the ester oil of the component (C).

The ester oils of the component (B) and the component (C) are carboxylate esters formed from an alcohol and a carboxylic acid, and may have a hydroxyl group or a carboxyl group within the structure.

The alcohol that constitutes the carboxylate ester is preferably an aliphatic alcohol or polyhydric alcohol having 1 to 22 carbon atoms.

The aliphatic alcohol that constitutes the carboxylate ester is preferably an alcohol having 5 to 20 carbon atoms, and specific examples thereof include isononanol, tridecanol, 2-ethylhexanol, cetanol, octyldodecanol, and isostearyl alcohol.

The polyhydric alcohol that constitutes the carboxylate ester is preferably a dihydric to octahydric alcohol, and is more preferably a dihydric to hexahydric alcohol having 2 to 16 carbon atoms, and specific examples thereof include propanediol, glycerol, diglycerol, neopentyl glycol, trimethylolpropane, pentaerythritol, and dipentaerythritol.

Methyl-branched isostearyl alcohol can be obtained by reducing isostearic acid which is widely used industrially. Isostearic acid, which is widely used industrially, is a carboxylic acid obtained by hydrogenating the monomer acid obtained as a by-product during the synthesis of dimer acid. The starting raw material in the dimer acid synthesis is mainly oleic acid, but may further contain linoleic acid and linolenic acid. As a result, the isostearic acid that is widely used industrially has an average of 1.3 methyl group branches on the main chain (see Fatty Acid Chemistry New Edition, second edition, edited by Keiichi Inaba and Jiro Hirano, published by Saiwai Shobo Co., Ltd., April 25, 1997 (page 224)).

On the other hand, 2-(1,3,3-trimethylbutyl)-5,7,7-trimethyloctanoic acid is generally called "isostearic acid".

Accordingly, a substituent derived from the alcohol obtained by reducing 2-(1,3,3-trimethylbutyl)-5,7,7-trimethyloctanoic acid is sometimes described using the expression "(having a 2-(1,3,3-trimethylbutyl)-5,7,7-trimethyloctyl structure in the aliphatic chain)" in the following description, in order to distinguish the alcohols derived from the above-mentioned two types of isostearic acid.

The carboxylic acid that constitutes the carboxylate ester is preferably a fatty acid having 1 to 22 carbon atoms, and the carboxylic acid may have a hydroxyl group or a carboxyl group. Specific examples thereof include caprylic acid, capric acid, 2-ethylhexanoic acid, isononanoic acid, palmitic acid, isostearic acid, lactic acid, malic acid, and hydroxystearic acid.

As described above for isostearyl alcohol, a plurality of compounds referred to as "isostearic acid" exist, despite having different structures. 2-(1,3,3-trimethylbutyl)-5,7,7-trimethyloctanoic acid or a substituent derived from this carboxylic acid is sometimes described using the expression "(having a 2-(1,3,3-trimethylbutyl)-5,7,7-trimethyloctyl structure in the aliphatic chain)" in the following description, in a similar manner to that described above for isostearyl alcohol.

There are no particular limitations on the ester oil of the component (B), and examples thereof include isononyl isononanoate [10.91], isotridecyl isononanoate [6.54], neopentyl glycol diethylhexanoate [6.28], glyceryl triethylhexanoate (triethylhexanoin) [3.79], glyceryl triisostearate (having a 2-(1,3,3-trimethylbutyl)-5,7,7-trimethyloctyl structure in the aliphatic chain) [3.78], diisostearyl malate (having a 2-(1,3,3-trimethylbutyl)-5,7,7-trimethyloctyl structure in the aliphatic chain) [3.72], pentaerythrityl tetraethylhexanoate [3.31], neopentyl glycol dicaprate [3.28], propanediol di(caprylate/caprate) [2.92], polyglyceryl-2 triisostearate (having a 2-(1,3,3-trimethylbutyl)-5,7,7-trimethyloctyl structure in the aliphatic chain) [2.68], cetyl ethylhexanoate [2.07], ethylhexyl palmitate [2.07], glyceryl tri(caprylate/caprate) [1.99], and dipentaerythrityl pentaisostearate (having a 2-(1,3,3-trimethylbutyl)-5,7,7-trimethyloctyl structure in the aliphatic chain) [1.89]. The number in the square brackets following each compound name is the dimethicone compatibility value calculated from the structural features of the ester oil using the above formula (I). One type of these compounds, or a combination of at least two types thereof may be used as ester oil of the component (B). Among these compounds, the ester oil of the component (B) is preferably isononyl isononanoate, isotridecyl isononanoate, neopentyl glycol diethylhexanoate, glyceryl triethylhexanoate, glyceryl triisostearate, diisostearyl malate, pentaerythrityl tetraethylhexanoate, neopentyl glycol dicaprate, propanediol di(caprylate/caprate), polyglyceryl-2 triisostearate, cetyl ethylhexanoate, glyceryl tri(caprylate/caprate), or dipentaerythrityl pentaisostearate. Although a hydroxyl group is present in the structure, diisostearyl malate is preferable as the ester oil of the component (B) in terms of compatibility with dimethicone and favorable compatibility with powders.

Commercially available ester oils may be used as the ester oil of the component (B). Examples of these commercially available ester oils include various ester oils manufactured by The Nisshin OilliO Group, Ltd., and specific examples thereof include isononyl isononanoate (product name: SALACOS 99), isotridecyl isononanoate (product name: SALACOS 913), neopentyl glycol diethylhexanoate (product name: SALACOS 525), triethylhexanoin (product name: T.I.O), triisostearin (product name: SALACOS 3318), diisostearyl malate (product name: COSMOL 222), pentaerythrityl tetraethylhexanoate (product name: SALACOS 5408), neopentyl glycol dicaprate (product name: ESTEMOL N-01), propanediol di(caprylate/caprate) (product name: SALACOS PR-85), polyglyceryl-2 triisostearate (product name: COSMOL 43N), cetyl ethylhexanoate (product name: SALACOS 816T), ethylhexyl palmitate (product name: SALACOS P-8), glyceryl tri(caprylate/caprate) (product name: O.D.O), and dipentaerythrityl pentaisostearate (product name: SALACOS DP-518N).

There are no particular limitations on the ester oil of the component (C), and examples thereof include propanediol diisostearate [1.71], trimethylolpropane triisostearate [1.36], pentaerythrityl tetraisostearate [1.04], polyglyceryl-2 tetraisostearate [0.82], polyglyceryl-2 triisostearate [0.48], and ethylhexyl hydroxystearate [-1.18]. The number in the square brackets following each compound name is the dimethicone compatibility value calculated from the structural features of the ester oil using the above formula (1). One type of these compounds, or a combination of at least two types thereof may be used as ester oil of the component (C). Ethylhexyl hydroxystearate is particularly preferred in terms of having a low dimethicone compatibility value, and separating efficiently from dimethicone.

Commercially available ester oils may be used as the ester oil of the component (C). Examples of these commercially available ester oils include various ester oils manufactured by The Nisshin OilliO Group, Ltd., and specific examples thereof include propanediol diisostearate (product name: SALACOS PR-17), trimethylolpropane triisostearate (product name: SALACOS 6318V), pentaerythrityl tetraisostearate (product name: SALACOS 5418V), polyglyceryl-2 tetraisostearate (product name: COSMOL 44V), polyglyceryl-2 triisostearate (product name: COSMOL 43V), and ethylhexyl hydroxystearate (product name: SALACOS EH).

The dimethicone compatibility value of each ester oil correlates with the compatibility when the component (A) and the ester oil are mixed in a mass ratio of 1:1. Specifically, among ester oils that are liquid at 25°C, ester oils having a dimethicone compatibility value of at least 1.75 exhibit compatibility when mixed with the component (A) in a mass ratio of 1:1. In contrast, among ester oils that are liquid at 25°C, ester oils having a dimethicone compatibility value of less than 1.75 do not exhibit compatibility when mixed with the component (A) in a mass ratio of 1:1. Accordingly, the liquid oily composition of the present embodiment can also be described by the following aspect without using dimethicone compatibility values.

In other words, the liquid oily composition of the present embodiment is a liquid oily composition which contains the following dimethicone (component (A2)) and ester oils (component (B2) and component (C2)) and is transparent and uniform at 25°C (compatibility 1), and in which the component (A2) separates, when 8 parts by mass of macadamia nut oil is added to 100 parts by mass of the liquid oily composition (compatibility 2).

Component (A2): one type, or at least two types, of dimethicone having a kinematic viscosity at 25°C within a range from 4 to 12 mm²/s.

Component (B2): one type, or at least two types, of ester oil that is liquid at 25°C, and exhibits compatibility when mixed with the component (A) in a mass ratio of 1:1 (mass ratio of component (A) : component (B2) = 1:1).

Component (C2): one type, or at least two types, of ester oil that is liquid at 25°C, and does not exhibit compatibility when mixed with the component (A) in a mass ratio of 1:1 (mass ratio of component (A) : component (C2) = 1:1).

The component (A2) is the same as the component (A).

Further, any ester oil that is liquid at 25°C and has a dimethicone compatibility value of at least 1.75, namely an ester oil of the component (B) described above, may be used as an ester oil of the component (B2).

Similarly, any ester oil that is liquid at 25°C and has a dimethicone compatibility value of less than 1.75, namely an ester oil of the component (C) described above, may be used as an ester oil of the component (C2).

The viscosity at 25°C of a mixture of the component (B2) and the component (C2) is preferably within a range from 10 to 1,500 mPa·s, more preferably from 30 to 1,200 mPa·s, even more preferably from 50 to 1,100 mPa·s, and most preferably from 100 to 1,000 mPa·s. By ensuring that the viscosity at 25°C of a mixture of the component (B2) and the component (C2) falls within the above-mentioned range, the liquid oily composition of the present embodiment is able to more readily form a silicone film when mixed with sebum. The above also applies to the viscosity at 25°C of a mixture of the component (B) and the component (C). The viscosity at 25°C of a mixture of the component (B2) and the component (C2) can be measured, for example, using a B-type viscometer, by selecting the rotor and rotational rate appropriately to achieve a reading within a range from 10 to 90, and then reading the value displayed 60 seconds after the start of rotation.

In the liquid oily composition of the present embodiment, the mass ratio ((B)/(C)) of the component (C) to the component (B) is preferably within a range from 0.32 to 18.00, and more preferably from 0.40 to 14.24.

Further, in the liquid oily composition of the present embodiment, the mass ratio ({(B)+(C)}/(A)) of the component (A) to the total mass of the component (B) and the component (C) is preferably within a range from 1.50 to 12.33, and more preferably from 2.08 to 11.05. By ensuring that the composition satisfies the conditions of the above mass ratio ((B)/(C)) and the above mass ratio ({(B)+(C)}/(A)), the liquid oily composition of the present embodiment is able to more readily form a silicone film when mixed with sebum. In other words, satisfying the conditions of the above mass ratio ((B)/(C)) and the above mass ratio ({(B)+(C)}/(A)) ensures a better inhibitory effect on secondary adhesion and superior water repellency with strength and persistence. The above also applies to the mass ratio ((B2)/(C2)) and the mass ratio ({(B2)+(C2)}/(A2)).

The combination and preferred blend proportions of the component (A), the component (B) and the component (C) in the liquid oily composition of the present embodiment are described below. In the following description, the sum of the component (A), the component (B) and the component (C) is deemed to be 100% by mass. Further, in the following examples, the value listed after the dimethicone indicates the kinematic viscosity of the dimethicone at 25°C. For example, "dimethicone 5 cs" means a dimethicone with a kinematic viscosity of 5 centistokes (5 cs). One centistoke is 1 mm²/s.

In the case where the component (A) is dimethicone 5 to 10 cs, the component (B) is glyceryl triethylhexanoate, and the component (C) is ethylhexyl hydroxystearate, the amount of the component (A) is preferably from 12.50 to 17.30% by mass, the amount of the component (B) is preferably from 31.40 to 41.20% by mass, and the amount of the component (C) is preferably from 45.00 to 53.00% by mass. In this case, the mass ratio ((B)/(C)) of the component (C) to the component (B) is preferably from 0.59 to 0.92, and the mass ratio ({(B)+(C)}/(A)) of the component (A) to the total mass of the component (B) and the component (C) is preferably from 4.78 to 7.00.

In the case where the component (A) is dimethicone 5 cs, the component (B) is glyceryl triethylhexanoate, and the component (C) is ethylhexyl hydroxystearate, the amount of the component (A) is preferably from 15.60 to 17.30% by mass, the amount of the component (B) is preferably from 31.40 to 34.70% by mass, and the amount of the component (C) is preferably from 48.00 to 53.00% by mass. In this case, the mass ratio ((B)/(C)) of the component (C) to the component (B) is preferably from 0.59 to 0.72, and the mass ratio ({(B)+(C)}/(A)) of the component (A) to the total mass of the component (B) and the component (C) is preferably from 4.78 to 5.41.

In the case where the component (A) is dimethicone 10 cs, the component (B) is isononyl isononanoate, and the component (C) is ethylhexyl hydroxystearate, the amount of the component (A) is preferably from 30.00 to 32.50% by mass, the amount of the component (B) is preferably from 30.00 to 32.50% by mass, and the amount of the component (C) is preferably from 35.00 to 40.00% by mass. In this case, the mass ratio ((B)/(C)) of the component (C) to the component (B) is preferably from 0.75 to 0.93, and the mass ratio ({(B)+(C)}/(A)) of the component (A) to the total mass of the component (B) and the component (C) is preferably from 2.08 to 2.33.

In the case where the component (A) is dimethicone 10 cs, the component (B) is isotridecyl isononanoate, and the component (C) is ethylhexyl hydroxystearate, the amount of the component (A) is preferably from 8.30 to 8.80% by mass, the amount of the component (B) is preferably from 24.70 to 26.20% by mass, and the amount of the component (C) is preferably from 65.00 to 67.00% by mass. In this case, the mass ratio ((B)/(C)) of the component (C) to the component (B) is preferably from 0.37 to 0.40, and the mass ratio ({(B)+(C)}/(A)) of the component (A) to the total mass of the component (B) and the component (C) is preferably from 10.36 to 11.05.

In the case where the component (A) is dimethicone 10 cs, the component (B) is neopentyl glycol diethylhexanoate, and the component (C) is ethylhexyl hydroxystearate, the amount of the component (A) is preferably from 9.00 to 9.50% by mass, the amount of the component (B) is preferably from 27.00 to 28.50% by mass, and the amount of the component (C) is preferably from 62.00 to 64.00% by mass. In this case, the mass ratio ((B)/(C)) of the component (C) to the component (B) is preferably from 0.42 to 0.46, and the mass ratio ({(B)+(C)}/(A)) of the component (A) to the total mass of the component (B) and the component (C) is preferably from 9.53 to 10.11.

In the case where the component (A) is dimethicone 10 cs, the component (B) is glyceryl triethylhexanoate, and the component (C) is ethylhexyl hydroxystearate, the amount of the component (A) is preferably from 12.50 to 13.80% by mass, the amount of the component (B) is preferably from 37.50 to 41.20% by mass, and the amount of the component (C) is preferably from 45.00 to 50.00% by mass. In this case, the mass ratio ((B)/(C)) of the component (C) to the component (B) is preferably from 0.75 to 0.92, and the mass ratio ({(B)+(C)}/(A)) of the component (A) to the total mass of the component (B) and the component (C) is preferably from 6.25 to 7.00.

In the case where the component (A) is dimethicone 10 cs, the component (B) is glyceryl triisostearate, and the component (C) is ethylhexyl hydroxystearate, the amount of the component (A) is preferably from 14.50 to 15.80% by mass, the amount of the component (B) is preferably from 43.50 to 47.20% by mass, and the amount of the component (C) is preferably from 37.00 to 42.00% by mass. In this case, the mass ratio ((B)/(C)) of the component (C) to the component (B) is preferably from 1.04 to 1.28, and the mass ratio ({(B)+(C)}/(A)) of the component (A) to the total mass of the component (B) and the component (C) is preferably from 5.33 to 5.90.

In the case where the component (A) is dimethicone 10 cs, the component (B) is pentaerythrityl tetraethylhexanoate, and the component (C) is ethylhexyl hydroxystearate, the amount of the component (A) is preferably from 16.30 to 17.50% by mass, the amount of the component (B) is preferably from 48.70 to 52.50% by mass, and the amount of the component (C) is preferably from 30.00 to 35.00% by mass. In this case, the mass ratio ((B)/(C)) of the component (C) to the component (B) is preferably from 1.39 to 1.75, and the mass ratio ({(B)+(C)}/(A)) of the component (A) to the total mass of the component (B) and the component (C) is preferably from 4.71 to 5.13.

In the case where the component (A) is dimethicone 10 cs, the component (B) is neopentyl glycol dicaprate, and the component (C) is ethylhexyl hydroxystearate, the amount of the component (A) is preferably from 16.30 to 18.00% by mass, the amount of the component (B) is preferably from 48.70 to 54.00% by mass, and the amount of the component (C) is preferably from 28.00 to 35.00% by mass. In this case, the mass ratio ((B)/(C)) of the component (C) to the component (B) is preferably from 1.39 to 1.93, and the mass ratio ({(B)+(C)}/(A)) of the component (A) to the total mass of the component (B) and the component (C) is preferably from 4.56 to 5.13.

In the case where the component (A) is dimethicone 10 cs, the component (B) is propanediol di(caprylate/caprate), and the component (C) is ethylhexyl hydroxystearate, the amount of the component (A) is preferably from 16.30 to 18.00% by mass, the amount of the component (B) is preferably from 48.70 to 54.00% by mass, and the amount of the component (C) is preferably from 28.00 to 35.00% by mass. In this case, the mass ratio ((B)/(C)) of the component (C) to the component (B) is preferably from 1.39 to 1.93, and the mass ratio ({(B)+(C)}/(A)) of the component (A) to the total mass of the component (B) and the component (C) is preferably from 4.56 to 5.13.

In the case where the component (A) is dimethicone 10 cs, the component (B) is polyglyceryl-2 triisostearate, and the component (C) is ethylhexyl hydroxystearate, the amount of the component (A) is preferably from 19.50 to 21.30% by mass, the amount of the component (B) is preferably from 58.50 to 63.70% by mass, and the amount of the component (C) is preferably from 15.00 to 22.00% by mass. In this case, the mass ratio ((B)/(C)) of the component (C) to the component (B) is preferably from 2.66 to 4.25, and the mass ratio ({(B)+(C)}/(A)) of the component (A) to the total mass of the component (B) and the component (C) is preferably from 3.69 to 4.13.

In the case where the component (A) is dimethicone 10 cs, the component (B) is cetyl ethylhexanoate, and the component (C) is ethylhexyl hydroxystearate, the amount of the component (A) is preferably from 11.80 to 13.00% by mass, the amount of the component (B) is preferably from 35.20 to 39.00% by mass, and the amount of the component (C) is preferably from 48.00 to 53.00% by mass. In this case, the mass ratio ((B)/(C)) of the component (C) to the component (B) is preferably from 0.66 to 0.81, and the mass ratio ({(B)+(C)}/(A)) of the component (A) to the total mass of the component (B) and the component (C) is preferably from 6.69 to 7.47.

In the case where the component (A) is dimethicone 10 cs, the component (B) is glyceryl tri(caprylate/caprate), and the component (C) is ethylhexyl hydroxystearate, the amount of the component (A) is preferably from 21.30 to 23.80% by mass, the amount of the component (B) is preferably from 63.70 to 71.20% by mass, and the amount of the component (C) is preferably from 5.00 to 15.00% by mass. In this case, the mass ratio ((B)/(C)) of the component (C) to the component (B) is preferably from 4.25 to 14.24, and the mass ratio ({(B)+(C)}/(A)) of the component (A) to the total mass of the component (B) and the component (C) is preferably from 3.20 to 3.69.

In the case where the component (A) is dimethicone 10 cs, the component (B) is dipentaerythrityl pentaisostearate, and the component (C) is ethylhexyl hydroxystearate, the amount of the component (A) is preferably from 20.00 to 22.00% by mass, the amount of the component (B) is preferably from 60.00 to 66.00% by mass, and the amount of the component (C) is preferably from 12.00 to 20.00% by mass. In this case, the mass ratio ((B)/(C)) of the component (C) to the component (B) is preferably from 3.00 to 5.50, and the mass ratio ({(B)+(C)}/(A)) of the component (A) to the total mass of the component (B) and the component (C) is preferably from 3.55 to 4.00.

In the case where the component (A) is dimethicone 10 cs, the component (B) is diisostearyl malate, and the component (C) is propanediol diisostearate, the amount of the component (A) is preferably from 12.50 to 16.30% by mass, the amount of the component (B) is preferably from 37.50 to 48.70% by mass, and the amount of the component (C) is preferably from 35.00 to 50.00% by mass. In this case, the mass ratio ((B)/(C)) of the component (C) to the component (B) is preferably from 0.75 to 1.39, and the mass ratio ({(B)+(C)}/(A)) of the component (A) to the total mass of the component (B) and the component (C) is preferably from 5.13 to 7.00.

In the case where the component (A) is dimethicone 10 cs, the component (B) is diisostearyl malate, and the component (C) is trimethylolpropane triisostearate, the amount of the component (A) is preferably from 18.80 to 19.50% by mass, the amount of the component (B) is preferably from 56.20 to 58.50% by mass, and the amount of the component (C) is preferably from 22.00 to 25.00% by mass. In this case, the mass ratio ((B)/(C)) of the component (C) to the component (B) is preferably from 2.25 to 2.66, and the mass ratio ({(B)+(C)}/(A)) of the component (A) to the total mass of the component (B) and the component (C) is preferably from 4.13 to 4.32.

In the case where the component (A) is dimethicone 10 cs, the component (B) is diisostearyl malate, and the component (C) is pentaerythrityl tetraisostearate, the amount of the component (A) is preferably from 18.80 to 20.00% by mass, the amount of the component (B) is preferably from 56.20 to 60.00% by mass, and the amount of the component (C) is preferably from 20.00 to 25.00% by mass. In this case, the mass ratio ((B)/(C)) of the component (C) to the component (B) is preferably from 2.25 to 3.00, and the mass ratio ({(B)+(C)}/(A)) of the component (A) to the total mass of the component (B) and the component (C) is preferably from 4.00 to 4.32.

In the case where the component (A) is dimethicone 10 cs, the component (B) is diisostearyl malate, and the component (C) is polyglyceryl-2 triisostearate, the amount of the component (A) is preferably from 20.00 to 21.00% by mass, the amount of the component (B) is preferably from 60.00 to 63.00% by mass, and the amount of the component (C) is preferably from 16.00 to 20.00% by mass. In this case, the mass ratio ((B)/(C)) of the component (C) to the component (B) is preferably from 3.00 to 3.94, and the mass ratio ({(B)+(C)}/(A)) of the component (A) to the total mass of the component (B) and the component (C) is preferably from 3.76 to 4.00.

In the case where the component (A) is dimethicone 10 cs, the component (B) is diisostearyl malate, and the component (C) is polyglyceryl-2 tetraisostearate, the amount of the component (A) is preferably from 18.80 to 20.00% by mass, the amount of the component (B) is preferably from 56.20 to 64.00% by mass, and the amount of the component (C) is preferably from 20.00 to 25.00% by mass. In this case, the mass ratio ((B)/(C)) of the component (C) to the component (B) is preferably from 2.25 to 3.20, and the mass ratio ({(B)+(C)}/(A)) of the component (A) to the total mass of the component (B) and the component (C) is preferably from 4.00 to 5.67.

In the case where the component (A) is dimethicone 10 cs, the component (B) is diisostearyl malate, and the component (C) is ethylhexyl hydroxystearate, the amount of the component (A) is preferably from 20.00 to 21.00% by mass, the amount of the component (B) is preferably from 60.00 to 63.00% by mass, and the amount of the component (C) is preferably from 16.00 to 20.00% by mass. In this case, the mass ratio ((B)/(C)) of the component (C) to the component (B) is preferably from 3.00 to 3.94, and the mass ratio ({ (B)+(C)}/(A)) of the component (A) to the total mass of the component (B) and the component (C) is preferably from 3.76 to 4.00.

### <Cosmetic Material>

Next is a description of a cosmetic material according to the present invention.

The cosmetic material of the present embodiment contains the liquid oily composition according to the embodiment described above. The cosmetic material may consist of the liquid oily composition, or may further contain one or more other components. When used as an oily component of a cosmetic material, the amount of the liquid oily composition relative to the total amount of the cosmetic material is preferably within a range from 5 to 60% by mass, more preferably from 10 to 50% by mass, and even more preferably from 10 to 40% by mass.

There are no particular limitations on the total amount of the component (A), the component (B) and the component (C) derived from the above liquid oily composition contained within the cosmetic material of the present embodiment, provided the cosmetic material exhibits compatibility 2. The total amount of the component (A), the component (B) and the component (C) relative to the total mass of the cosmetic material of the present embodiment may be, for example, 5% by mass or more, and is preferably within a range from 10 to 60% by mass, more preferably from 15 to 50% by mass, even more preferably from 20 to 45% by mass, and may be 100% by mass.

The cosmetic material according to the present invention may have an external appearance that is transparent, semi-transparent or emulsified (cloudy), and may have any of various forms such as a liquid or a cream.

There are no particular limitations on the cosmetic material using the liquid oily composition according to the present invention, and examples thereof include makeup cosmetics such as foundations, sunscreen cosmetics, and skincare cosmetics such as emulsions, creams, essences and skin lotions.

### (Component (D))

The cosmetic material of the present embodiment may further contain a powder as a component (D).

Any of the powders typically used in cosmetic materials may be used as the powder of the component (D). There are no particular limitations on the powder of the component (D), and extender pigments, color pigments, and pearl pigments and the like may be used.

Furthermore, there are no particular limitations on the shape of the powder of the component (D), and any shape including spherical, plate-like, needle-like, granular, spindle-like, flake-like or amorphous shapes may be used.

There are no particular limitations on the extender pigments, and examples thereof include inorganic pigments such as silicic acid, silicic anhydride, magnesium silicate, talc, sericite, mica, kaolin, clay, bentonite, bismuth oxychloride, zirconium oxide, magnesium oxide, zinc oxide, aluminum oxide, calcium sulfate, barium sulfate, magnesium sulfate, calcium carbonate and magnesium carbonate, and composite powders thereof; organic powders such as polyamide, polyester, polypropylene, polystyrene, polyurethane, nylon, silicone resins, vinyl resins, urea resins, phenol resins, silicon resins, acrylic resins, melamine resins, epoxy resins, polycarbonate resins, divinylbenzene-styrene copolymers, silk powder, cellulose and N-ε-lauroyl-L-lysine, and composites powders thereof; and composite powders of the above inorganic powders and the above organic powders.

There are no particular limitations on the color pigments, and examples thereof include metal oxides such as titanium oxide, zinc oxide, yellow iron oxide, bengala, black iron oxide, red iron oxide, Prussian blue, ultramarine, chromium oxide and chromium hydroxide; metal complexes such as manganese violet and cobalt titanate; inorganic pigments such as carbon black; organic pigments such as tar dyes and lake pigments; and natural colorants such as carmine.

There are no particular limitations on the pearl pigments, and pearl pigments in which mica, synthetic phlogopite or the like is coated with a colorant such as titanium oxide, iron oxide, silicon oxide, Prussian blue, chromium oxide, carmine, an organic pigment or the like may be used, for example. These powders may be used after carrying out any of various surface treatments, such as a water repellency treatment or a water and oil repellency treatment, by known methods.

A sunscreen cosmetic material (cosmetic material for preventing sunburn) can also be produced by including a powder that exhibits an ultraviolet-shielding effect such as a metal oxide powder as the powder of the component (D).

Although there are no particular limitations on the metal oxide powder that exhibits an ultraviolet-shielding effect, it is preferable, for example, that the average particle size be within a range from 10 to 100 nm, because the generation of white powder residue when applied to the skin can be suppressed.

There are no particular limitations on the metal oxide powder that exhibits an ultraviolet-shielding effect, and examples thereof include titanium oxide, zinc oxide, zirconium oxide and cerium oxide, and one type of these powders or a combination of at least two types thereof may be used.

Further, there are no particular limitations on the shape, and examples thereof include spherical, needle-like, spindle-like, plate-like, and flake-like shapes. The powder may also be treated with an inorganic compound such as silica or alumina to reduce the surface activity.

In those cases where the cosmetic material of the present embodiment includes the component (D), the appropriate amount of the component (D) relative to the total mass of the cosmetic material varies depending on the type of cosmetic material, but is preferably within a range from 1 to 90% by mass, more preferably from 1 to 60% by mass, and even more preferably from 1 to 30% by mass. For example, if the cosmetic material of the present embodiment is a water-in-oil emulsion foundation, an emulsion eye-shadow or a sunscreen, the amount of the component (D) within the cosmetic material is preferably within a range from 1 to 30% by mass.

### (Component (E) and Component (F))

The cosmetic material of the present embodiment may further contain water as the component (E) and a surfactant as the component (F).

The cosmetic material of the present embodiment can be formed as an emulsion cosmetic material by including these components. The cosmetic material of the present embodiment may be a water-in-oil emulsion cosmetic material (W/O emulsion cosmetic material) or an oil-in-water emulsion cosmetic material (O/W emulsion cosmetic material).

There are no particular limitations on the water used as the component (E), and any of ion-exchanged water, distilled water, water derived from fruit, deep-sea water, or the like may be used, for example.

There are no particular limitations on the amount of the component (E) in the cosmetic material of the present embodiment containing the component (E), and the amount may be adjusted appropriately in view of the type of cosmetic material or the like. The amount of the component (E) within the cosmetic material of the present embodiment is preferably within a range from 1 to 80% by mass, more preferably from 10 to 70% by mass, and even more preferably from 20 to 60% by mass.

Any surfactant typically available in cosmetic products may be used as the surfactant of the component (F). Among them, nonionic surfactants such as polyoxyethylene-modified silicones, polyglycerol fatty acid esters, alkyl polyoxyethylenes, polyoxyethylene fatty acid esters, and polyoxyethylene hydrogenated castor oil are preferred.

There are no particular limitations on the amount of the component (F) in the cosmetic material of the present embodiment containing the component (F), and the amount may be adjusted appropriately in view of the type of cosmetic material or the like. The amount of the component (F) within the cosmetic material of the present embodiment is preferably within a range from 0.01 to 10% by mass, more preferably from 0.05 to 5% by mass, and even more preferably from 0.1 to 5% by mass.

The cosmetic material according to the present invention may further include additives typically added to cosmetic materials, provided the effects of the present invention are not impaired. Examples of the additives include antioxidants, antioxidant assistants, preservatives, ultraviolet absorbers, polyhydric alcohols, water-soluble polymers, pH regulators, inorganic salts or salts of organic acids, chelate agents, vitamins, organic solvents, fragrances, and various extracts. One type of these additives may be used alone, or a combination of at least two types thereof may be used.

Examples of the antioxidants include, but are not limited to, oil-soluble vitamin C derivatives, tocopherols and derivatives and salts thereof, dibutylhydroxytoluene, butylhydroxyanisole, and gallate esters. One type of these antioxidants may be used alone, or a combination of at least two types thereof may be used.

Examples of the antioxidant assistants include, but are not limited to, phosphoric acid, citric acid, maleic acid, malonic acid, succinic acid, fumaric acid, cephalin, hexametaphosphate, phytic acid, and ethylenediaminetetraacetic acid. One type of these antioxidant assistants may be used alone, or a combination of at least two types thereof may be used.

Examples of the preservatives include, but are not limited to, methylparaben, ethylparaben, butylparaben and phenoxyethanol. One type of these preservatives may be used alone, or a combination of at least two types thereof may be used.

Examples of the ultraviolet absorbers include, but are not limited to, benzoic acid-based ultraviolet absorbers such as para-aminobenzoic acid (hereinafter, abbreviated as PABA), PABA monoglycerol ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester; anthranilic acid-based ultraviolet absorbers such as homomenthyl-N-acetylanthranilate; salicylic acid-based ultraviolet absorbers such as amyl salicylate, menthyl salicylate, homomenthyl salicylate, octyl salicylate, phenyl salicylate, benzyl salicylate, and p-isopropanol phenyl salicylate; cinnamic acid-based ultraviolet absorbers such as octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate and glycerylmono-2-ethylhexanoyl-diparamethoxy cinnamate; benzophenone-based ultraviolet absorbers such as 2,4-dihydroxybenzophenone, 2,2'-dihydroxy-4-methoxybenzophenone, 2.2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone; as well as 3-(4'-methylbenzylidene)-d,l-camphor, 3-benzylidene-d,l-camphor, urocanic acid, ethyl urocanate, 2-phenyl-5-methylbenzoxazole, 2,2'-hydroxy-5-methylphenylbenzotriazole, 2-(2'-hydroxy-5'-t-octylphenyl)benzotriazole, 2-(2'-hydroxy-5'-methylphenyl)benzotriazole, dibenzalazine, dianisoylmethane, 4-methoxy-4'-t butyldibenzoylmethane, 5-(3,3-dimethyl-2-norbornylidene)-3-pentan-2-one, 2,4,6-trianilino-p-(carbo-2'-ethylhexyl-1'-oxy)-1,3,5-triazine, 4-tert-butyl-4'-methoxydibenzoylmethane, hexyl ethylaminohydroxybenzoyl benzoate, ethylhexyltriazine, t-butylmethoxybenzoylmethane, bisethylhexyloxyphenol methoxyphenyltriazine, diethylhexyl butamido triazone, and oxybenzone-3. One type of these ultraviolet absorbers may be used alone, or a combination of at least two types thereof may be used.

Examples of the polyhydric alcohols include, but are limited to, propylene glycol (1,2-propanediol), 1,3-propanediol, 1,3-butylene glycol (1,3-butanediol), pentylene glycol (1,2-pentanediol), neopentylene glycol (2,2-dimethyl-1,3-propanediol), isoprene glycol (3-methyl-1,3-butanediol), dipropylene glycol, glycerol, diglycerol, polyglycerol, polyethylene glycol, pentaerythritol, dipentaerythritol, sorbitol, and sorbitan. One type of these polyhydric alcohols may be used alone, or a combination of at least two types thereof may be used.

There are no particular limitations on the water-soluble polymer, and examples thereof include natural water-soluble polymers, semi-synthetic water-soluble polymers, and synthetic water-soluble polymers. One type of these water-soluble thickeners may be included alone in the cosmetic material according to the present invention, or a combination of at least two types thereof may be included in the cosmetic material according to the present invention.

There are no particular limitations on the natural water-soluble polymers, and examples thereof include plant-based polymers such as agar, glucomannan, gum arabic, tragacanth gum, galactan, guar gum, carob gum, karaya gum, carrageenan, pectin, quince seeds (marmelo), algae colloid (algae extract), and starch (rice, corn, potato or wheat); microorganism-derived polymers such as xanthan gum, dextran, succinoglucan, and pullulan; and animal-based polymers such as collagen, casein, albumin, and gelatin.

There are no particular limitations on the semi-synthetic water-soluble polymers, and examples thereof include starch-based polymers such as carboxymethyl starch and methylhydroxypropyl starch; cellulose-based polymers such as methyl cellulose, nitrocellulose, methyl hydroxypropyl cellulose, sodium cellulose sulphate, hydroxypropyl cellulose, carboxymethyl cellulose, sodium carboxymethyl cellulose, crystalline cellulose, and cellulose powder; and alginic acid-based polymers such as sodium alginate and propylene glycol alginate.

There are no particular limitations on the synthetic water-soluble polymers, and examples thereof include vinyl-based polymers such as polyvinyl alcohol, polyvinyl methyl ether, polyvinylpyrrolidone, and carboxy vinyl polymer; polyoxyethylene-based polymers such as polyethylene glycol 20,000, 40,000 and 60,000; polyoxyethylene polyoxypropylene copolymer-copolymer-based polymers; acrylic-based polymers such as sodium polyacrylate, polyethyl acrylate and polyacrylamide; as well as polyethyleneimine and cationic polymers.

There are no particular limitations on the pH regulators, and examples thereof include edetic acid, edetate disodium, citric acid, sodium citrate, sodium hydroxide, potassium hydroxide, and triethanolamine. One type of these pH regulators may be used alone, or a combination of at least two types thereof may be used.

There are no particular limitations on the inorganic salts, and examples thereof include sodium chloride, potassium chloride, magnesium chloride, sodium sulphate, potassium sulphate, and magnesium sulphate. Examples of the salts of organic acids include citric acid, malic acid, tartaric acid and salts thereof, ascorbic acid and salts thereof, and ascorbic acid derivatives and salts thereof.

There are no particular limitations on the chelate agents, and examples thereof include edetate disodium, salts of edetic acid, and hydroxyethanediphosphonic acid. One type of these chelate agents may be used alone, or a combination of at least two types thereof may be used.

There are no particular limitations on the vitamins, and examples thereof include vitamin A, vitamin B1, vitamin B2, vitamin B6, vitamin E, vitamin K, derivatives thereof, pantothenic acid and derivatives thereof, and biotin.

There are no particular limitations on the extracts, and examples thereof include plant extracts such as aloe vera, witch hazel, hamamelis, cucumber, lemon, lavender, and rose.

### EXAMPLES

The present invention is described below in further detail based on specific examples. However, the present invention is not limited by the content of the examples described below.

### [1. Evaluations of Liquid Oily Compositions]

Table 1 lists compounds and corresponding abbreviations as the component (B) and the component (C).

Table 2 shows the test results of compatibility with dimethicones having various kinematic viscosities and the dimethicone compatibility value of the compounds as the component (B) and the component (C).

Table 3 shows the dimethicone compatibility value and the molecular structure factors used in the calculation of the dimethicone compatibility value.

The compatibility of the dimethicone and the component (B) or the component (C) was evaluated in accordance with the below-mentioned "Preparation of Liquid Oily Compositions and Evaluation of Compatibility 1".

As a result, it was confirmed that the ester oils which exhibited compatibility with dimethicone had a dimethicone compatibility value of at least 1.75. In contrast, it was confirmed that the ester oils which lacked compatibility with dimethicone had a dimethicone compatibility value of less than 1.75.

**[Table 1]**

| Table 1 | | |
|---|---|---|
| Component (B) | B1 | Isononyl isononanoate |
| | B2 | Isotridecyl isononanoate |
| | B3 | Neopentyl glycol diethylhexanoate |
| | B4 | Glyceryl triethylhexanoate |
| | B5 | Glyceryl triisostearate |
| | B6 | Diisostearyl malate |
| | B7 | Pentaerythrityl tetraethylhexanoate |
| | B8 | Neopentyl glycol dicaprate |
| | B9 | Propanediol di(caprylate/caprate) |
| | B10 | Polyglyceryl-2 triisostearate |
| | B11 | Cetyl ethylhexanoate |
| | B12 | Ethylhexyl palmitate |
| | B13 | Glyceryl tri(caprylate/caprate) |
| | B14 | Dipentaerythrityl pentaisostearate |
| Component (C) | C1 | Propanediol diisostearate |
| | C2 | Trimethylolpropane triisostearate |
| | C3 | Pentaerythrityl tetraisostearate |
| | C4 | Polyglyceryl-2 tetraisostearate |
| | C5 | Polyglyceryl-2 triisostearate |
| | C6 | Ethylhexyl hydroxystearate |

**[Table 2]**

| Table 2 | Compatibility of dimethicones of various viscosities (including component (A)) and components (B) in mixtures with a mass ratio of 1:1 | | | | | | |
|---|---|---|---|---|---|---|---|
| | Component abbreviation | Dimethicone compatibility value | Type of dimethicone | | | | |
| | | | Dimethicone 5 cs | Dimethicone 10 cs | Dimethicone 20 cs | Dimethicone 100 cs | Dimethicone 1,000,000 cs |
| Component (B) | B1 | 10.91 | compatible | compatible | compatible | compatible | compatible |
| | B2 | 6.54 | compatible | compatible | compatible | compatible | compatible |
| | B3 | 6.28 | compatible | compatible | compatible | compatible | compatible |
| | B4 | 3.79 | compatible | compatible | compatible | compatible | not compatible |
| | B5 | 3.78 | compatible | compatible | compatible | not compatible | not compatible |
| | B6 | 3.72 | compatible | compatible | compatible | not compatible | not compatible |
| | B7 | 3.31 | compatible | compatible | compatible | not compatible | not compatible |
| | B8 | 3.28 | compatible | compatible | compatible | not compatible | not compatible |
| | B9 | 2.92 | compatible | compatible | compatible | not compatible | not compatible |
| | B10 | 2.68 | compatible | compatible | not compatible | not compatible | not compatible |
| | B11 | 2.07 | compatible | compatible | compatible | not compatible | not compatible |
| | B12 | 2.07 | compatible | compatible | compatible | not compatible | not compatible |
| | B13 | 1.99 | compatible | compatible | not compatible | not compatible | not compatible |
| | B14 | 1.89 | compatible | compatible | not compatible | not compatible | not compatible |
| Component (C) | C1 | 1.71 | not compatible | not compatible | not compatible | not compatible | not compatible |
| | C2 | 1.36 | not compatible | not compatible | not compatible | not compatible | not compatible |
| | C3 | 1.04 | not compatible | not compatible | not compatible | not compatible | not compatible |
| | C4 | 0.82 | not compatible | not compatible | not compatible | not compatible | not compatible |
| | C5 | 0.48 | not compatible | not compatible | not compatible | not compatible | not compatible |
| | C6 | -1.18 | not compatible | not compatible | not compatible | not compatible | not compatible |

**[Table 3]**

| Table 3 | Component abbreviation | Dimethicone compatibility value | Molecular weight | Number of tertiary carbons | Number of quaternary carbons | Number of carbon branches | Number of hydroxyl groups | Number of ester groups | Number of ether groups |
|---|---|---|---|---|---|---|---|---|---|
| Component (B) | B1 | 10.91 | 284 | 2 | 2 | 6 | 0 | 1 | 0 |
| | B2 | 6.54 | 341 | 3 | 1 | 5 | 0 | 1 | 0 |
| | B3 | 6.28 | 357 | 2 | 1 | 4 | 0 | 2 | 0 |
| | B4 | 3.79 | 471 | 3 | 0 | 3 | 0 | 3 | 0 |
| | B5 | 3.78 | 891 | 9 | 6 | 21 | 0 | 3 | 0 |
| | B6 | 3.72 | 639 | 6 | 4 | 14 | 1 | 2 | 0 |
| | B7 | 3.31 | 641 | 4 | 1 | 6 | 0 | 4 | 0 |
| | B8 | 3.28 | 413 | 0 | 1 | 2 | 0 | 2 | 0 |
| | B9 | 2.92 | 331 | 0 | 0 | 0 | 0 | 2 | 0 |
| | B10 | 2.68 | 966 | 9 | 6 | 21 | 1 | 3 | 1 |
| | B11 | 2.07 | 368 | 1 | 0 | 1 | 0 | 1 | 0 |
| | B12 | 2.07 | 368 | 1 | 0 | 1 | 0 | 1 | 0 |
| | B13 | 1.99 | 491 | 0 | 0 | 0 | 0 | 3 | 0 |
| | B14 | 1.89 | 1624 | 15 | 12 | 39 | 1 | 5 | 1 |
| Component (C) | C1 | 1.71 | 608 | 2.6 | 0 | 2.6 | 0 | 2 | 0 |
| | C2 | 1.36 | 934 | 3.9 | 1 | 5.9 | 0 | 3 | 0 |
| | C3 | 1.04 | 1202 | 5.2 | 1 | 7.2 | 0 | 4 | 0 |
| | C4 | 0.82 | 1232 | 5.2 | 0 | 5.2 | 0 | 4 | 1 |
| | C5 | 0.48 | 966 | 3.9 | 0 | 3.9 | 1 | 3 | 1 |
| | C6 | -1.18 | 412 | 1 | 0 | 1 | 1 | 1 | 0 |

The dimethicones used were all manufactured by Shin-Etsu Chemical Co., Ltd., wherein the dimethicone under the product name of "KF-96A-1 cs" (hereinafter, abbreviated as "dimethicone 1 cs") was used as a dimethicone having a kinematic viscosity at 25°C of 1 mm²/s, the dimethicone under the product name of "KF-96L-2cs" (hereinafter, abbreviated as "dimethicone 2 cs") was used as a dimethicone having a kinematic viscosity of 2 mm²/s, the dimethicone under the product name of "KF-96A-5cs" (hereinafter, abbreviated as "dimethicone 5 cs") was used as a dimethicone having a kinematic viscosity of 5 mm²/s, the dimethicone under the product name of "KF-96A-10cs" (hereinafter, abbreviated as "dimethicone 10 cs") was used as a dimethicone having a kinematic viscosity of 10 mm²/s, the dimethicone under the product name of "KF-96A-20cs" (hereinafter, abbreviated as "dimethicone 20 cs") was used as a dimethicone having a kinematic viscosity of 20 mm²/s, the dimethicone under the product name of "KF-96A-100cs" (hereinafter, abbreviated as "dimethicone 100 cs") was used as a dimethicone having a kinematic viscosity of 100 mm²/s, and the dimethicone under the product name of "KF-96A-1,000,000cs" (hereinafter, abbreviated as "dimethicone 1,000,000 cs") was used as a dimethicone having a kinematic viscosity of 1,000,000 mm²/s. In the following description, dimethicones that do not correspond with the definition of the component (A) are sometimes referred to as the component (A').

All of the compounds used as the component (B) were commercially available products manufactured by The Nisshin OilliO Group, Ltd., and isononyl isononanoate (product name: SALACOS 99), isotridecyl isononanoate (product name: SALACOS 913), neopentyl glycol diethylhexanoate (product name: SALACOS 525), glyceryl triethylhexanoate (product name: T.I.O), glyceryl triisostearate (product name: SALACOS 3318), diisostearyl malate (product name: COSMOL 222), pentaerythrityl tetraethylhexanoate (product name: SALACOS 5408), neopentyl glycol dicaprate (product name: ESTEMOL N-01), propanediol di(caprylate/caprate) (product name: SALACOS PR-85), polyglyceryl-2 triisostearate (product name: COSMOL 43N), cetyl ethylhexanoate (product name: SALACOS 816T), ethylhexyl palmitate (product name: SALACOS P-8), glyceryl tri(caprylate/caprate) (product name: O.D.O), and dipentaerythrityl pentaisostearate (product name: SALACOS DP-518N) were used.

All of the compounds used as the component (C) were commercially available products manufactured by The Nisshin OilliO Group, Ltd., and propanediol diisostearate (product name: SALACOS PR-17), trimethylolpropane triisostearate (product name: SALACOS 6318V), pentaerythrityl tetraisostearate (product name: SALACOS 5418V), polyglyceryl-2 tetraisostearate (product name: COSMOL 44V), polyglyceryl-2 triisostearate (product name: COSMOL 43V), and ethylhexyl hydroxystearate (product name: SALACOS EH) were used.

Examples of the liquid oily composition according to one embodiment of the present invention are described below.

Table 4 to Table 14 show the constitutions (% by mass) of liquid oily compositions, and the evaluation results of those liquid oily compositions. The numerical values in the component columns of Table 4 to Table 14 indicate % by mass values.

### (Raw Materials of Liquid Oily Compositions)

Iron oxide (product name: OTS-2 RED R-516) manufactured by DAITO KASEI KOGYO CO., LTD., was used as the pigment.

Macadamia nut oil (product name: Pure Macadamia Nut Oil) manufactured by Yokozeki-Oil-Fat-Industries-Co., LTD., was used as the macadamia nut oil.

### [Method for Preparation of Liquid Oily Composition]

The component (A) or component (A'), the component (B) and the component (C) of each of the constitutions shown in Tables 4 to 14 were weighed into the same screw-top vial, and were then shaken and mixed thoroughly to prepare a liquid oily composition.

The compatibility 1, the compatibility 2, the viscosity of a mixture of the component (B) and the component (C), the inhibitory effect on secondary adhesion of a pigment, the strength of the water repellency, and the persistence of the water repellency of the obtained liquid oily compositions in Tables 4 to 7 were investigated by the below-mentioned methods, and an overall evaluation (in terms of the inhibitory effect on secondary adhesion of a pigment, and the strength and persistence of water repellency) was also carried out.

Furthermore, the compatibility 1 and the compatibility 2 of each of the obtained liquid oily compositions in Tables 8 to 14 were investigated by the below-mentioned methods.

### [Method for Evaluation of Compatibility 1]

The screw-top vial in which the prepared liquid oily composition was put was left to stand at 25°C for five minutes. Subsequently, the external appearance of the liquid oily composition was confirmed by visual inspection, and the compatibility was evaluated in accordance with the below-mentioned evaluation criteria of compatibility 1. This compatibility of the prepared liquid oily compositions was defined as compatibility 1.

### [Evaluation Criteria of Compatibility 1]

S: It was confirmed that the composition was compatible (had compatibility) from the external appearance at 25°C which was transparent and uniform without generating separation, cloudiness or turbidity.

IS: It was confirmed that the composition was not compatible (lacked compatibility) from the external appearance at 25°C in which separation, cloudiness or turbidity was generated.

The liquid oily composition according to the present invention requires that the mixture of the component (A), the component(B) and the component (C) is transparent and uniform, namely, is evaluated as "S" in terms of the compatibility 1.

### [Method for Evaluating Compatibility 2]

Eight parts by mass of macadamia nut oil were added to 100 parts by mass of the liquid oily composition which had been evaluated in terms of the compatibility 1, and the mixture was shaken and mixed thoroughly. The screw-top vial was then left to stand at 25°C for five minutes. Subsequently, the external appearance of the liquid oily composition was confirmed by visual inspection, and the compatibility was evaluated in accordance with the below-mentioned evaluation criteria of the compatibility 2. This compatibility of the liquid oily composition following addition of macadamia nut oil to the liquid oily composition was defined as compatibility 2.

### [Evaluation Criteria of Compatibility 2]

S: It was confirmed that the composition was compatible (had compatibility) from the external appearance which was transparent and uniform without generating separation, cloudiness or turbidity.

IS: It was confirmed that the composition was not compatible (lacked compatibility) from the external appearance in which separation, cloudiness or turbidity was generated.

The liquid oily composition according to the present invention requires that when eight parts by mass of macadamia nut oil is added to 100 parts by mass of the liquid oily composition, the resulting mixture is not compatible, namely, evaluated as "IS" in terms of the compatibility 2.

### [Evaluation of Compatibility 1 and Compatibility 2]

When a liquid oily composition is evaluated as "S" in terms of the compatibility 1 and evaluated as "IS" in terms of the compatibility 2, the liquid oily composition exhibits the effects of the present invention.

### [Viscosity of Mixture of Component (B) and Component (C)]

The viscosity at 25°C of a mixture of the component (B) and the component (C) was measured using a B-type viscometer. **In** the viscosity measurement, the rotor and rotational rate were selected appropriately to achieve a reading within a range from 10 to 90, and the value displayed 60 seconds after the start of rotation was then read and converted to a viscosity value.

### [Method for Evaluating Inhibitory Effect on Secondary Adhesion of Pigment]

(I) The component (A), the component (B) and the component (C) were mixed as shown in Table 4, Table 5, Table 6 or Table 7 to obtain a liquid oily composition. 5% by mass of a pigment was then added relative to the total mass of the obtained liquid oily composition. Iron oxide was used as the pigment. Subsequently, the mixture was placed in an ultrasonic cleaning device, and subjected to an ultrasonic treatment for 60 minutes with occasional shaking and stirring of the mixture.
(II) A 3 cm × 3 cm region on the inside of a forearm was washed to remove any sebum. Three µL of macadamia nut oil was then applied to the prepared region as a substitute sebum for the removed sebum.
(III) Nine µL of the liquid oily composition containing the pigment prepared in (I) was then applied on top of the region to which the macadamia nut oil had been applied and left to stand for three minutes.
(IV) Tissue paper was placed on top to cover the applied region, and was pressed lightly from above. Subsequently, the tissue paper was removed, and the degree of transfer of the pigment to the tissue paper was evaluated.

### [Calculation of Secondary Adhesion Area]

The degree of transfer of the pigment to the tissue paper was evaluated by photographing the tissue from a height of 25 cm above the target (the tissue), and then subjecting the captured image to image processing. The image processing conditions involved using the image processing software "imageJ" to trim the image to include the entire transfer region and center the transfer region, subsequently carrying out binarization processing, and then calculating the area of the black portions. In the binarization processing, the captured image (color image) was first converted to a gray scale image by 8-bit conversion. Subsequently, the binarization processing was carried out by converting the gray scale image to black at portions where the image values were within a range from 0 to 195 and to white at portions where the image values were within a range from 196 to 255.

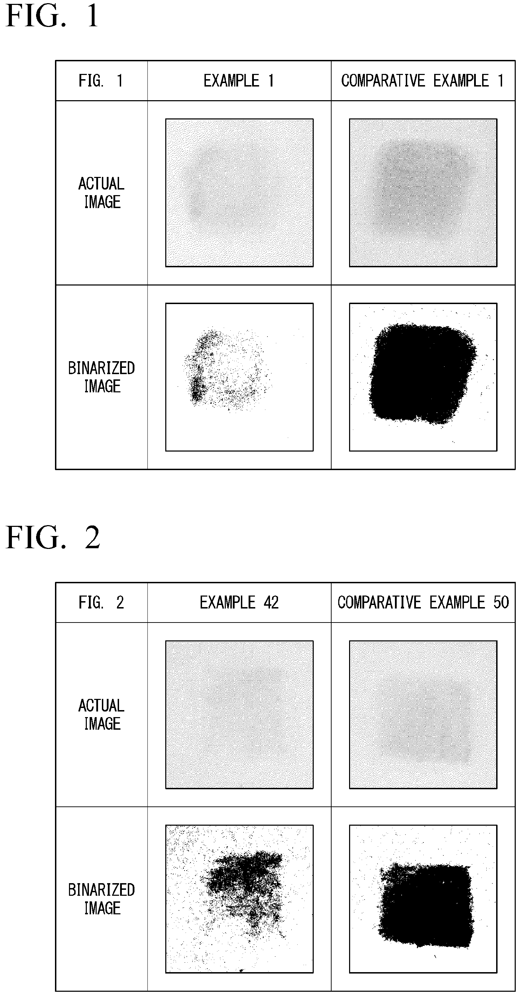

The captured photographs (actual images) of Example 1 and Comparative Example 1, as well as the images obtained by subjecting those photographs to binarization processing are shown in FIG. 1.

### [Evaluation Criteria of Inhibition on Secondary Adhesion of Pigment]

The inhibitory effect on secondary adhesion of a pigment was evaluated using the proportion of the detected area of the black portion calculated in the above-mentioned "Calculation of Secondary Adhesion Area".
A: The detected area was less than 25%.
B: The detected area was at least 25% but less than 28%.
C: The detected area was at least 28% but less than 30%.
D: The detected area was 30% or higher.

In the case of the grade D evaluated when the detected area was 30% or higher, no inhibitory effect was deemed to be exhibited.

### [Method for Evaluating Strength of Water Repellency]

(I) Three µL of macadamia nut oil was applied to a 50 mm × 50 mm PMMA plate.
(II) Nine µL of the liquid oily composition prepared as shown in Table 3 or Table 4 was applied to the PMMA plate.
(III) Subsequently, after standing for three minutes, 20 µL of ion-exchanged water was dripped onto the coating film applied to the plate, the plate was then inclined, and the angle at which the water droplet started to move was measured.

### [Evaluation Criteria of Strength of Water Repellency]

A: The water droplet started to move at a plate inclination angle of less than 40°.
B: The water droplet started to move at a plate inclination angle of at least 40° but less than 41°.
C: The water droplet started to move at a plate inclination angle of at least 42° but less than 43°.
D: The water droplet did not move even at a plate inclination angle of 43° or higher.

### [Method for Evaluating Persistence of Water Repellency]

(I) A PMMA plate prepared in the same manner as described above in the "Method for Evaluating Strength of Water Repellency " was immersed for ten minutes in ion-exchanged water held at 40°C, and then the plate was dried naturally.
(II) Subsequently, 20 µL of ion-exchanged water was dripped onto the coating film applied to the plate, the plate was then inclined, and the angle at which the water droplet started to move was measured.

### [Evaluation Criteria of Persistence of Water Repellency]

A: The water droplet started to move at a plate inclination angle of less than 34°.
B: The water droplet started to move at a plate inclination angle of at least 34° but less than 35°.
C: The water droplet started to move at a plate inclination angle of at least 35° but less than 36°.
D: The water droplet did not move even at a plate inclination angle of 36° or higher.

### [Overall Evaluation of Inhibitory Effect on Secondary Adhesion of Pigment, and Strength and Persistence of Water Repellency]

Each of the grades of the liquid oily compositions obtained by evaluating the inhibitory effect on the secondary adhesion of a pigment, the strength of water repellency and the persistence of water repellency was converted to a numerical score by assigning four points to the grade A, three points to the grade B, two points to the grade C, and one point to the grade D, and the sum of the numerical scores was used as an overall evaluation score. However, liquid oily compositions evaluated as the grade D in terms of at least one of the inhibitory effect on secondary adhesion of a pigment, the strength of water repellency and the persistence of water repellency were deemed to lack the effects of the present invention.

**[Table 4]**

| Table 4 | | Constitution (% by mass) of liquid oily compositions, and evaluation results | | | | | |
|---|---|---|---|---|---|---|---|
| Components (raw materials) | | Dimethicone compatibility value | Example 1 | Example 2 | Example 3 | Example 4 | Example 5 |
| Component (A) | Dimethicone 10 cs | - | 20.00 | 21.00 | 15.00 | 16.00 | 24.00 |
| Component (B) | Diisostearyl malate | 2.94 | 60.00 | 63.00 | 60.00 | 64.00 | 0 |
| | Isononyl isononanoate | 10.91 | 0 | 0 | 0 | 0 | 36.0 |
| Component (C) | Ethylhexyl hydroxystearate | -1.18 | 20.00 | 16.00 | 0 | 0 | 0 |
| | Polyglyceryl-2 tetraisostearate | 0.83 | 0 | 0 | 25.00 | 20.00 | 40.00 |
| Total | | - | 100.00 | 100.00 | 100.00 | 100.00 | 100.00 |
| (B)/(C) | | - | 3.00 | 3.94 | 2.40 | 3.20 | 0.90 |
| {(B)+(C)}/(A) | | - | 4.00 | 3.76 | 5.67 | 5.25 | 3.17 |
| Compatibility 1 | | - | S | S | S | S | S |
| Compatibility 2 | | - | IS | IS | IS | IS | IS |
| Viscosity of mixture of component (B) and component (C) (25°C, mPa·s) | | - | 550 | 725 | 950 | 1100 | 45 |
| Evaluation results | Inhibition on secondary adhesion | - | A | A | B | C | A |
| | Strength of water repellency | - | A | A | B | B | C |
| | Persistence of water repellency | - | A | A | B | B | C |
| | Overall evaluation | - | 12 | 12 | 9 | 8 | 8 |

**[Table 5]**

| Table 5 | | Constitution (% by mass) of liquid oily compositions, and evaluation results | | | | | | | |
|---|---|---|---|---|---|---|---|---|---|
| Components (raw materials) | | Dimethicone compatibility value | Comparative Example 1 | Comparative Example 2 | Comparative Example 3 | Comparative Example 4 | Comparative Example 5 | Comparative Example 6 | Comparative Example 7 |
| Component (A) | Dimethicone 10 cs | - | 20.00 | 20.00 | 18.75 | 22.50 | 14.00 | 17.00 | 16.20 |
| Component (B) | Diisostearyl malate | 2.94 | 80.00 | 0 | 56.25 | 67.50 | 56.00 | 68.00 | 64.80 |
| | Isononyl isononanoate | 10.91 | 0 | 0 | 0 | 0 | 0 | 0 | 0 |
| Component (C) | Ethylhexyl hydroxystearate | -1.18 | 0 | 80.00 | 25.00 | 10.00 | 0 | 0 | 0 |
| | Polyglyceryl-2 tetraisostearate | 0.83 | 0 | 0 | 0 | 0 | 30.00 | 15.00 | 19.00 |
| Total | | - | 100.00 | 100.00 | 100.00 | 100.00 | 100.00 | 100.00 | 100.00 |
| (B)/(C) | | - | - | 0 | 2.25 | 6.75 | 1.87 | 4.53 | 3.41 |
| {(B)+(C)}/(A) | | - | 4.00 | 4.00 | 4.33 | 3.44 | 6.14 | 4.88 | 5.17 |
| Compatibility 1 | | - | s | IS | IS | S | IS | s | s |
| Compatibility 2 | | - | S | IS | IS | S | IS | S | S |
| Viscosity of mixture of component (B) and component (C) (25°C, mPa·s) | | - | 3100 | 90 | 425 | 1025 | 800 | 1376 | 1376 |
| Evaluation results | Inhibition on secondary adhesion | - | D | - | - | D | - | D | D |
| | Strength of water repellency | - | D | - | - | D | - | D | D |
| | Persistence of water repellency | - | D | - | - | D | - | D | D |
| | Overall evaluation | - | 3 | - | - | 3 | - | 3 | 3 |

**[Table 6]**

| Table 6 | | Constitution (% by mass) of liquid oily compositions, and evaluation results | | | | | |
|---|---|---|---|---|---|---|---|
| Components (raw materials) | | Dimethicone compatibility value | Example 6 | Example 7 | Comparative Example 8 | Comparative Example 9 | Comparative Example 10 |
| Component (A') | Dimethicone 1 cs | - | 0 | 0 | 0 | 0 | 0 |
| Component (A') | Dimethicone 2 cs | - | 0 | 0 | 0 | 0 | 0 |
| Component (A) | Dimethicone 5 cs | - | 17.30 | 15.60 | 40.00 | 13.30 | 0 |
| Component (A') | Dimethicone 20 cs | - | 0 | 0 | 0 | 0 | 20.00 |
| Component (B) | Glyceryl triethylhexanoate | 3.79 | 34.70 | 31.40 | 20.00 | 26.70 | 60.00 |
| Component (C) | Ethylhexyl hydroxystearate | -1.18 | 48.00 | 53.00 | 40.00 | 60.00 | 20.00 |
| Total | | - | 100.00 | 100.00 | 100.00 | 100.00 | 100.00 |
| (B)/(C) | | - | 0.72 | 0.59 | 0.50 | 0.45 | 3.00 |
| {(B)+(C)}/(A) or (A') | | - | 4.78 | 5.41 | 1.50 | 6.52 | 4.00 |
| Compatibility 1 | | - | S | S | S | IS | S |
| Compatibility 2 | | - | IS | IS | S | IS | IS |
| Viscosity of mixture of component (B) and component (C) (25°C, mPa·s) | | - | - | - | - | - | - |
| Evaluation results | Inhibition on secondary adhesion | - | A | A | - | - | D |
| | Strength of water repellency | - | A | A | - | - | B |
| | Persistence of water repellency | - | A | A | - | - | B |
| | Overall evaluation | - | 12.00 | 12.00 | - | - | 7.00 |

**[Table 7]**

| Table 7 | | Constitution (% by mass) of liquid oily compositions, and evaluation results | | | | | |
|---|---|---|---|---|---|---|---|
| Components (raw materials) | | Dimethicone compatibility value | Comparative Example 11 | Comparative Example 12 | Comparative Example 13 | Comparative Example 14 | Comparative Example 15 |
| Component (A') | Dimethicone 1 cs | - | 0 | 0 | 0 | 17.30 | 0 |
| Component (A') | Dimethicone 2 cs | - | 0 | 0 | 0 | 0 | 17.30 |
| Component (A) | Dimethicone 5 cs | - | 0 | 0 | 0 | 0 | 0 |
| Component (A') | Dimethicone 20 cs | - | 18.0 | 21.25 | 17.50 | 0 | 0 |
| Component (B) | Glyceryl triethylhexanoate | 3.79 | 54.00 | 63.75 | 52.50 | 34.70 | 34.70 |
| Component (C) | Ethylhexyl hydroxystearate | -1.18 | 28.00 | 15.00 | 30.00 | 48.00 | 48.00 |
| Total | | - | 100.00 | 100.00 | 100.00 | 100.00 | 100.00 |
| (B)/(C) | | - | 1.93 | 4.25 | 1.75 | 0.72 | 0.72 |
| {(B)+(C)}/(A) or (A') | | - | 4.56 | 3.71 | 4.71 | 4.78 | 4.78 |
| Compatibility 1 | | - | S | S | IS | S | S |
| Compatibility 2 | | - | IS | S | IS | S | S |
| Viscosity of mixture of component (B) and component (C) (25°C, mPa·s) | | - | - | - | - | - | - |
| Evaluation results | Inhibition on secondary adhesion | - | D | - | - | - | - |
| | Strength of water repellency | - | B | - | - | - | - |
| | Persistence of water repellency | - | B | - | - | - | - |
| | Overall evaluation | - | 7.00 | - | - | - | - |

**[Table 8]**

| Table 8 | | Constitution (% by mass) of liquid oily compositions, and evaluation results | | | | | | | | | | |
|---|---|---|---|---|---|---|---|---|---|---|---|---|
| Components (raw materials) | | Dimethicone compatibility value | Example 8 | Example 9 | Example 10 | Example 11 | Example 12 | Example 13 | Example 14 | Example 15 | Example 16 | Example 17 |
| Component (A) | Dimethicone 10 cs | - | 32.50 | 30.00 | 8.80 | 8.30 | 9.50 | 9.00 | 13.80 | 12.50 | 15.80 | 14.50 |
| Component (B) | B1 | 10.91 | 32.50 | 30.00 | 0 | 0 | 0 | 0 | 0 | 0 | 0 | 0 |
| | B2 | 6.54 | 0 | 0 | 26.20 | 24.70 | 0 | 0 | 0 | 0 | 0 | 0 |
| | B3 | 6.28 | 0 | 0 | 0 | 0 | 28.50 | 27.00 | 0 | 0 | 0 | 0 |
| | B4 | 3.79 | 0 | 0 | 0 | 0 | 0 | 0 | 41.20 | 37.50 | 0 | 0 |
| | B5 | 3.78 | 0 | 0 | 0 | 0 | 0 | 0 | 0 | 0 | 47.20 | 43.50 |
| | B7 | 3.31 | 0 | 0 | 0 | 0 | 0 | 0 | 0 | 0 | 0 | 0 |
| | B8 | 3.28 | 0 | 0 | 0 | 0 | 0 | 0 | 0 | 0 | 0 | 0 |
| | B9 | 2.92 | 0 | 0 | 0 | 0 | 0 | 0 | 0 | 0 | 0 | 0 |
| | B10 | 2.68 | 0 | 0 | 0 | 0 | 0 | 0 | 0 | 0 | 0 | 0 |
| | B11 | 2.07 | 0 | 0 | 0 | 0 | 0 | 0 | 0 | 0 | 0 | 0 |
| | B13 | 1.99 | 0 | 0 | 0 | 0 | 0 | 0 | 0 | 0 | 0 | 0 |
| | B14 | 1.89 | 0 | 0 | 0 | 0 | 0 | 0 | 0 | 0 | 0 | 0 |
| Component (C) | C6 | -1.18 | 35.00 | 40.00 | 65.00 | 67.00 | 62.00 | 64.00 | 45.00 | 50.00 | 37.00 | 42.00 |
| Total | | | 100.00 | 100.00 | 100.00 | 100.00 | 100.00 | 100.00 | 100.00 | 100.00 | 100.00 | 100.00 |
| (B)/(C) | | - | 0.93 | 0.75 | 0.40 | 0.37 | 0.46 | 0.42 | 0.92 | 0.75 | 1.28 | 1.04 |
| {(B)+(C) }/(A) | | - | 2.08 | 2.33 | 10.36 | 11.05 | 9.53 | 10.11 | 6.25 | 7.00 | 5.33 | 5.90 |
| Compatibility 1 | | - | S | S | S | S | S | S | S | S | S | S |
| Compatibility 2 | | - | IS | IS | IS | IS | IS | IS | IS | IS | IS | IS |

**[Table 9]**

| Table 9 | | Constitution (% by mass) of liquid oily compositions, and evaluation results | | | | | | | | | | |
|---|---|---|---|---|---|---|---|---|---|---|---|---|
| Components (raw materials) | | Dimethicone compatibility value | Example 18 | Example 19 | Example 20 | Example 21 | Example 22 | Example 23 | Example 24 | Example 25 | Example 26 | Example 27 |
| Component (A) | Dimethicone 10 cs | - | 17.50 | 16.30 | 18.00 | 16.30 | 18.00 | 16.30 | 21.30 | 19.50 | 13.00 | 11.80 |
| Component (B) | B1 | 10.91 | 0 | 0 | 0 | 0 | 0 | 0 | 0 | 0 | 0 | 0 |
| | B2 | 6.54 | 0 | 0 | 0 | 0 | 0 | 0 | 0 | 0 | 0 | 0 |
| | B3 | 6.28 | 0 | 0 | 0 | 0 | 0 | 0 | 0 | 0 | 0 | 0 |
| | B4 | 3.79 | 0 | 0 | 0 | 0 | 0 | 0 | 0 | 0 | 0 | 0 |
| | B5 | 3.78 | 0 | 0 | 0 | 0 | 0 | 0 | 0 | 0 | 0 | 0 |
| | B7 | 3.31 | 52.50 | 48.70 | 0 | 0 | 0 | 0 | 0 | 0 | 0 | 0 |
| | B8 | 3.28 | 0 | 0 | 54.00 | 48.70 | 0 | 0 | 0 | 0 | 0 | 0 |
| | B9 | 2.92 | 0 | 0 | 0 | 0 | 54.00 | 48.70 | 0 | 0 | 0 | 0 |
| | B10 | 2.68 | 0 | 0 | 0 | 0 | 0 | 0 | 63.70 | 58.50 | 0 | 0 |
| | B11 | 2.07 | 0 | 0 | 0 | 0 | 0 | 0 | 0 | 0 | 39.00 | 35.20 |
| | B13 | 1.99 | 0 | 0 | 0 | 0 | 0 | 0 | 0 | 0 | 0 | 0 |
| | B14 | 1.89 | 0 | 0 | 0 | 0 | 0 | 0 | 0 | 0 | 0 | 0 |
| Component (C) | C6 | -1.18 | 30.00 | 35.00 | 28.00 | 35.00 | 28.00 | 35.00 | 15.00 | 22.00 | 48.00 | 53.00 |
| Total | | | 100.00 | 100.00 | 100.00 | 100.00 | 100.00 | 100.00 | 100.00 | 100.00 | 100.00 | 100.00 |
| (B)/(C) | | - | 1.75 | 1.39 | 1.93 | 1.39 | 1.93 | 1.39 | 4.25 | 2.66 | 0.81 | 0.66 |
| {(B)+(C) }/(A) | | - | 4.71 | 5.13 | 4.56 | 5.13 | 4.56 | 5.13 | 3.69 | 4.13 | 6.69 | 7.47 |
| Compatibility 1 | | - | S | S | S | S | S | S | S | S | S | S |
| Compatibility 2 | | - | IS | IS | IS | IS | IS | IS | IS | IS | IS | IS |

**[Table 10]**

| Table 10 | | Constitution (% by mass) of liquid oily compositions, and evaluation results | | | | | | | | | | |
|---|---|---|---|---|---|---|---|---|---|---|---|---|
| Components (raw materials) | | Dimethicone compatibility value | Example 28 | Example 29 | Example 30 | Example 31 | Comparative Example 16 | Comparative Example 17 | Comparative Example 18 | Comparative Example 19 | Comparative Example 20 | Comparative Example 21 |
| Component (A) | Dimethicone 10 cs | - | 23.80 | 21.30 | 22.00 | 20.00 | 35.00 | 25.00 | 10.00 | 7.50 | 12.50 | 7.50 |
| Component (B) | B1 | 10.91 | 0 | 0 | 0 | 0 | 35.00 | 25.00 | 0 | 0 | 0 | 0 |
| | B2 | 6.54 | 0 | 0 | 0 | 0 | 0 | 0 | 30.00 | 22.50 | 0 | 0 |
| | B3 | 6.28 | 0 | 0 | 0 | 0 | 0 | 0 | 0 | 0 | 37.50 | 22.50 |
| | B4 | 3.79 | 0 | 0 | 0 | 0 | 0 | 0 | 0 | 0 | 0 | 0 |
| | B5 | 3.78 | 0 | 0 | 0 | 0 | 0 | 0 | 0 | 0 | 0 | 0 |
| | B7 | 3.31 | 0 | 0 | 0 | 0 | 0 | 0 | 0 | 0 | 0 | 0 |
| | B8 | 3.28 | 0 | 0 | 0 | 0 | 0 | 0 | 0 | 0 | 0 | 0 |
| | B9 | 2.92 | 0 | 0 | 0 | 0 | 0 | 0 | 0 | 0 | 0 | 0 |
| | B10 | 2.68 | 0 | 0 | 0 | 0 | 0 | 0 | 0 | 0 | 0 | 0 |
| | B11 | 2.07 | 0 | 0 | 0 | 0 | 0 | 0 | 0 | 0 | 0 | 0 |
| | B13 | 1.99 | 71.20 | 63.70 | 0 | 0 | 0 | 0 | 0 | 0 | 0 | 0 |
| | B14 | 1.89 | 0 | 0 | 66.00 | 60.00 | 0 | 0 | 0 | 0 | 0 | 0 |
| Component (C) | C6 | -1.18 | 5.00 | 15.00 | 12.00 | 20.00 | 30.00 | 50.00 | 60.00 | 70.00 | 50.00 | 70.00 |
| Total | | | 100.00 | 100.00 | 100.00 | 100.00 | 100.00 | 100.00 | 100.00 | 100.00 | 100.00 | 100.00 |
| (B)/(C) | | - | 14.24 | 4.25 | 5.50 | 3.00 | 1.17 | 0.50 | 0.50 | 0.32 | 0.75 | 0.32 |
| {(B)+(C) }/(A) | | - | 3.20 | 3.69 | 3.55 | 4.00 | 0.86 | 2.00 | 9.00 | 12.33 | 7.00 | 12.33 |
| Compatibility 1 | | - | S | S | S | S | S | IS | S | IS | S | IS |
| Compatibility 2 | | - | IS | IS | IS | IS | S | IS | S | IS | S | IS |

**[Table 11]**

| Table 11 | | Constitution (% by mass) of liquid oily compositions, and evaluation results | | | | | | | | | | |
|---|---|---|---|---|---|---|---|---|---|---|---|---|
| Components (raw materials) | | Dimethicone compatibility value | Comparative Example 22 | Comparative Example 23 | Comparative Example 24 | Comparative Example 25 | Comparative Example 26 | Comparative Example 27 | Comparative Example 28 | Comparative Example 29 | Comparative Example 30 | Comparative Example 31 |
| Component (A) | Dimethicone 10 cs | - | 15.00 | 10.00 | 17.50 | 12.50 | 18.80 | 15.00 | 18.80 | 15.00 | 20.00 | 15.00 |
| Component (B) | B1 | 10.91 | 0 | 0 | 0 | 0 | 0 | 0 | 0 | 0 | 0 | 0 |
| | B2 | 6.54 | 0 | 0 | 0 | 0 | 0 | 0 | 0 | 0 | 0 | 0 |
| | B3 | 6.28 | 0 | 0 | 0 | 0 | 0 | 0 | 0 | 0 | 0 | 0 |
| | B4 | 3.79 | 45.00 | 30.00 | 0 | 0 | 0 | 0 | 0 | 0 | 0 | 0 |
| | B5 | 3.78 | 0 | 0 | 52.50 | 37.50 | 0 | 0 | 0 | 0 | 0 | 0 |
| | B7 | 3.31 | 0 | 0 | 0 | 0 | 56.20 | 45.00 | 0 | 0 | 0 | 0 |
| | B8 | 3.28 | 0 | 0 | 0 | 0 | 0 | 0 | 56.20 | 45.00 | 0 | 0 |
| | B9 | 2.92 | 0 | 0 | 0 | 0 | 0 | 0 | 0 | 0 | 60.00 | 45.00 |
| | B10 | 2.68 | 0 | 0 | 0 | 0 | 0 | 0 | 0 | 0 | 0 | 0 |
| | B11 | 2.07 | 0 | 0 | 0 | 0 | 0 | 0 | 0 | 0 | 0 | 0 |
| | B13 | 1.99 | 0 | 0 | 0 | 0 | 0 | 0 | 0 | 0 | 0 | 0 |
| | B14 | 1.89 | 0 | 0 | 0 | 0 | 0 | 0 | 0 | 0 | 0 | 0 |
| Component (C) | C6 | -1.18 | 40.00 | 60.00 | 30.00 | 50.00 | 25.00 | 40.00 | 25.00 | 40.00 | 20.00 | 40.00 |
| Total | | | 100.00 | 100.00 | 100.00 | 100.00 | 100.00 | 100.00 | 100.00 | 100.00 | 100.00 | 100.00 |
| (B)/(C) | | - | 1.13 | 0.50 | 1.75 | 0.75 | 2.25 | 1.13 | 2.25 | 1.13 | 3.00 | 1.13 |
| {(B)+(C)}/(A) | | - | 5.67 | 9.00 | 4.71 | 7.00 | 4.32 | 5.67 | 4.32 | 5.67 | 4.00 | 5.67 |
| Compatibility 1 | | - | S | IS | S | IS | S | IS | S | IS | S | IS |
| Compatibility 2 | | - | S | IS | S | IS | S | IS | S | IS | S | IS |

**[Table 12]**

| Table 12 | | Constitution (% by mass) of liquid oily compositions, and evaluation results | | | | | | | | |
|---|---|---|---|---|---|---|---|---|---|---|
| Components (raw materials) | | Dimethicone compatibility value | Comparative Example 32 | Comparative Example 33 | Comparative Example 34 | Comparative Example 35 | Comparative Example 36 | Comparative Example 37 | Comparative Example 38 | Comparative Example 39 |
| Component (A) | Dimethicone 10 cs | - | 22.50 | 18.80 | 15.00 | 10.00 | 24.00 | 20.00 | 22.50 | 18.80 |
| Component (B) | B1 | 10.91 | 0 | 0 | 0 | 0 | 0 | 0 | 0 | 0 |
| | B2 | 6.54 | 0 | 0 | 0 | 0 | 0 | 0 | 0 | 0 |
| | B3 | 6.28 | 0 | 0 | 0 | 0 | 0 | 0 | 0 | 0 |
| | B4 | 3.79 | 0 | 0 | 0 | 0 | 0 | 0 | 0 | 0 |
| | B5 | 3.78 | 0 | 0 | 0 | 0 | 0 | 0 | 0 | 0 |
| | B7 | 3.31 | 0 | 0 | 0 | 0 | 0 | 0 | 0 | 0 |
| | B8 | 3.28 | 0 | 0 | 0 | 0 | 0 | 0 | 0 | 0 |
| | B9 | 2.92 | 0 | 0 | 0 | 0 | 0 | 0 | 0 | 0 |
| | B10 | 2.68 | 67.50 | 56.20 | 0 | 0 | 0 | 0 | 0 | 0 |
| | B11 | 2.07 | 0 | 0 | 45.00 | 30.00 | 0 | 0 | 0 | 0 |
| | B13 | 1.99 | 0 | 0 | 0 | 0 | 72.00 | 60.00 | 0 | 0 |
| | B14 | 1.89 | 0 | 0 | 0 | 0 | 0 | 0 | 67.50 | 56.20 |
| Component (C) | C6 | -1.18 | 10.00 | 25.00 | 40.00 | 60.00 | 4.00 | 20.00 | 10.00 | 25.00 |
| Total | | | 100.00 | 100.00 | 100.00 | 100.00 | 100.00 | 100.00 | 100.00 | 100.00 |
| (B)/(C) | | - | 6.75 | 2.25 | 1.13 | 0.50 | 18.00 | 3.00 | 6.75 | 2.25 |
| {(B)+(C) }/(A) | | - | 3.44 | 4.32 | 5.67 | 9.00 | 3.17 | 4.00 | 3.44 | 4.32 |
| Compatibility 1 | | - | S | IS | S | IS | S | IS | S | IS |
| Compatibility 2 | | - | S | IS | S | IS | S | IS | S | IS |

**[Table 13]**

| Table 13 | | Constitution (% by mass) of liquid oily compositions, and evaluation results | | | | | | | | | | |
|---|---|---|---|---|---|---|---|---|---|---|---|---|
| Components (raw materials) | | Dimethicone compatibility value | Example 32 | Example 33 | Example 34 | Example 35 | Example 36 | Example 37 | Example 38 | Example 39 | Example 40 | Example 41 |
| Component (A) | Dimethicone 10 cs | - | 16.30 | 12.50 | 19.50 | 18.80 | 20.00 | 18.80 | 20.00 | 18.80 | 21.00 | 20.00 |
| Component (B) | B6 | 3.72 | 48.70 | 37.50 | 58.50 | 56.20 | 60.00 | 56.20 | 60.00 | 56.20 | 63.00 | 60.00 |
| Component (C) | C1 | 1.71 | 35.00 | 50.00 | 0 | 0 | 0 | 0 | 0 | 0 | 0 | 0 |
| | C2 | 1.36 | 0 | 0 | 22.00 | 25.00 | 0 | 0 | 0 | 0 | 0 | 0 |
| | C3 | 1.04 | 0 | 0 | 0 | 0 | 20.00 | 25.00 | 0 | 0 | 0 | 0 |
| | C4 | 0.82 | 0 | 0 | 0 | 0 | 0 | 0 | 20.00 | 25.00 | 0 | 0 |
| | C5 | 0.48 | 0 | 0 | 0 | 0 | 0 | 0 | 0 | 0 | 16.00 | 20.00 |
| Total | | | 100.00 | 100.00 | 100.00 | 100.00 | 100.00 | 100.00 | 100.00 | 100.00 | 100.00 | 100.00 |
| (B)/(C) | | - | 1.39 | 0.75 | 2.66 | 2.25 | 3.00 | 2.25 | 3.00 | 2.25 | 3.94 | 3.00 |
| {(B)+(C)}/(A) | | - | 5.13 | 7.00 | 4.13 | 4.32 | 4.00 | 4.32 | 4.00 | 4.32 | 3.76 | 4.00 |
| Compatibility 1 | | - | S | S | S | S | S | S | S | S | S | S |
| Compatibility 2 | | - | IS | IS | IS | IS | IS | IS | IS | IS | IS | IS |

**[Table 14]**

| Table 14 | | Constitution (% by mass) of liquid oily compositions, and evaluation results | | | | | | | | | | |
|---|---|---|---|---|---|---|---|---|---|---|---|---|
| Components (raw materials) | | Dimethicone compatibility value | Comparative Example 40 | Comparative Example 41 | Comparative Example 42 | Comparative Example 43 | Comparative Example 44 | Comparative Example 45 | Comparative Example 46 | Comparative Example 47 | Comparative Example 48 | Comparative Example 49 |
| Component (A) | Dimethicone 10 cs | - | 17.50 | 10.00 | 20.00 | 17.50 | 21.30 | 17.50 | 21.30 | 17.50 | 22.50 | 18.70 |
| Component (B) | B6 | 3.72 | 52.50 | 30.00 | 60.00 | 52.50 | 63.70 | 52.50 | 63.70 | 52.50 | 67.50 | 56.30 |
| Component (C) | C1 | 1.71 | 30.00 | 60.00 | 0 | 0 | 0 | 0 | 0 | 0 | 0 | 0 |
| | C2 | 1.36 | 0 | 0 | 20.00 | 30.00 | 0 | 0 | 0 | 0 | 0 | 0 |
| | C3 | 1.04 | 0 | 0 | 0 | 0 | 15.00 | 30.00 | 0 | 0 | 0 | 0 |
| | C4 | 0.82 | 0 | 0 | 0 | 0 | 0 | 0 | 15.00 | 30.00 | 0 | 0 |
| | C5 | 0.48 | 0 | 0 | 0 | 0 | 0 | 0 | 0 | 0 | 10.00 | 25.00 |
| Total | | | 100.00 | 100.00 | 100.00 | 100.00 | 100.00 | 100.00 | 100.00 | 100.00 | 100.00 | 100.00 |
| (B)/(C) | | - | 1.75 | 0.50 | 3.00 | 1.75 | 4.25 | 1.75 | 4.25 | 1.75 | 6.75 | 2.25 |
| {(B)+(C)}/(A) | | - | 4.71 | 9.00 | 4.00 | 4.71 | 3.69 | 4.71 | 3.69 | 4.71 | 3.44 | 4.35 |
| Compatibility 1 | | - | S | IS | S | IS | S | IS | S | IS | S | IS |
| Compatibility 2 | | - | S | IS | S | IS | S | IS | S | IS | S | IS |

It was confirmed form the results in Table 4 to Table 7 that the liquid oily compositions of Examples 1 to 7, in which the dimethicone 5 cs or dimethicone 10 cs was used as the component (A), and which were evaluated as S in terms of the compatibility 1 (compatible) and evaluated as IS in terms of the compatibility 2 (not compatible), exhibited an excellent inhibitory effect on secondary adhesion of a pigment, and had excellent water repellency with strength and persistence. It is surmised that these results were obtained by separating the component (A) when applied to the skin and mixed with sebum, thereby forming a silicone film on the surface.

In contrast, the liquid oily composition of Comparative Example 1, in which the dimethicone 10 cs as was used the component (A) but the component (C) was not contained, evaluated as S in terms of the compatibility 1 and compatibility 2 (compatible), and as a result, the inhibitory effect on secondary adhesion of a pigment and the strength and persistence of the water repellency were poor. Furthermore, the liquid oily composition of Comparative Example 2, in which the dimethicone 10 cs was used as the component (A) but the component (B) was not contained, was evaluated as IS in terms of the compatibility 1 and compatibility 2 (not compatible). The liquid oily composition of Comparative Example 2 was not uniform, and therefore evaluations of the inhibitory effect on secondary adhesion and the strength and persistence of water repellency were not carried out.

Furthermore, although the dimethicone 10 cs was used as the component (A) in the liquid oily composition of Comparative Example 3 and Comparative Example 5, the liquid oily compositions were evaluated as IS in terms of the compatibility 1 and compatibility 2 (not compatible). The liquid oily compositions of Comparative Example 3 and the Comparative Example 5 were not uniform, and therefore evaluations of the inhibitory effect on secondary adhesion and the strength and persistence of water repellency were not carried out.

Further, although the dimethicone 10 cs was used as the component (A) in each of the liquid oily compositions of Comparative Example 4, Comparative Example 6 and Comparative Example 7, the liquid oily compositions were evaluated as S in terms of the compatibility 1 and compatibility 2 (compatible), and the inhibitory effect on secondary adhesion of a pigment and the strength and persistence of water repellency were poor.

Although the dimethicone 5 cs was used as the component (A) in the liquid oily composition of Comparative Example 8, the liquid oily composition was evaluated as S in terms of the compatibility 1 and compatibility 2 (compatible). Based on the evaluation results of Comparative Example 4, Comparative Example 6 and Comparative Example 7, which obtained the same compatibility evaluations, there is a high probability that the inhibitory effect on secondary adhesion of a pigment and the strength and persistence of water repellency of the liquid oily composition of Comparative Example 8 were also poor.

Further, although the dimethicone 5 cs was used as the component (A) in the liquid oily composition of Comparative Example 9, the liquid oily composition was evaluated as IS in terms of the compatibility 1 and compatibility 2 (not compatible). The liquid oily composition of Comparative Example 9 was not uniform, and therefore evaluations of the inhibitory effect on secondary adhesion and the strength and persistence of water repellency were not carried out.

Although the liquid oily compositions of Comparative Examples 10 and 11, in which the dimethicone 20 cs was used, was evaluated as S in terms of the compatibility 1 (compatible) and was evaluated as IS in terms of the compatibility 2 (not compatible), the inhibitory effect on secondary adhesion of a pigment was poor. Based on this result, it was evident that the dimethicone 20 cs could not be used as the dimethicone of the component (A).

Further, the liquid oily composition of Comparative Example 12, in which the dimethicone 20 cs was used, was evaluated as S in terms of the compatibility 1 and compatibility 2 (compatible). Based on the evaluation results of Comparative Example 4, Comparative Example 6 and Comparative Example 7, which obtained the same compatibility evaluations, there is a high probability that the inhibitory effect on secondary adhesion of a pigment and the strength and persistence of water repellency of the liquid oily composition of Comparative Example 12 were also poor.

Furthermore, the liquid oily composition of Comparative Example 13, in which the dimethicone 20 cs was used, was evaluated as IS in terms of the compatibility 1 and compatibility 2 (not compatible). The liquid oily composition of Comparative Example 13 was not uniform, and therefore evaluations of the inhibitory effect on secondary adhesion and the strength and persistence of water repellency were not carried out.

The liquid oily composition of Comparative Example 14, in which the constitution was the same as that of Example 6 except that the dimethicone 1 cs was used instead of the dimethicone 10 cs, and the liquid oily compositions of Comparative Example 15, in which the constitution was the same as that of Example 6 except that dimethicone 2 cs was used instead of the dimethicone 10 cs, were evaluated as S in terms of the compatibility 1 and compatibility 2 (compatible). Based on the evaluation results of Comparative Example 4, Comparative Example 6 and Comparative Example 7, which obtained the same compatibility evaluations, there is a high probability that the inhibitory effect on secondary adhesion of a pigment and the strength and persistence of water repellency of the liquid oily compositions of Comparative Examples 14 and 15 were also poor. Based on these results, it was evident that the dimethicone 1 cs and the dimethicone 2 cs could not be used as the dimethicone of the component (A).

Based on the above results, it was evident that in those cases where the compatibility 1 was evaluated as "S" and the compatibility 2 was evaluated as "IS", the separated dimethicone formed a silicone film, thereby exhibiting the effects of the present invention.

The liquid oily compositions of Examples 8 to 41 shown in Table 8 to Table 10 and Table 13 were not evaluated in terms of the inhibitory effect on secondary adhesion of a pigment and the strength and persistence of water repellency. However, each of the liquid oily compositions contained the dimethicone 10 cs as the component (A) and was evaluated as S in terms of the compatibility 1 (compatible) and evaluated as IS in terms of the compatibility 2 (not compatible), and therefore based on the evaluation results of Examples 1 to 5, in which the dimethicone 10 cs was used as the component (A) and the same compatibility evaluations were obtained, there is a high probability that the liquid oily compositions of Examples 8 to 41 also had an excellent inhibitory effect on secondary adhesion of pigment as well as excellent water repellency with strength and persistence.

Further, the liquid oily compositions of Comparative Examples 16 to 49 shown in Table 10 to Table 12 and Table 14 were also not evaluated in terms of the inhibitory effect on secondary adhesion of a pigment and the strength and persistence of water repellency. However, there is a high probability that the inhibitory effect on secondary adhesion of a pigment, the strength of water repellency, and the persistence of water repellency of the liquid oily compositions of the comparative examples in which the compatibility 1 and the compatibility 2 were evaluated as S (compatible) were poor, based on the evaluation results of Comparative Example 4, Comparative Example 6 and Comparative Example 7, in which the dimethicone 10 cs was used as the component (A) and the same compatibility evaluations were obtained.

Furthermore, the liquid oily compositions of the comparative examples in Table 10 to Table 12 and Table 14 that were evaluated as IS in terms of the compatibility 1 and the compatibility 2 (not compatible) were not uniform, and therefore evaluations of the inhibitory effect on secondary adhesion and the strength and persistence of water repellency were not carried out.

### [2. Evaluation of Cosmetic Materials]

Next, cosmetic materials each containing the liquid oily composition, according to another embodiment of the present invention, were investigated. Following preparation of cosmetic materials of Example 42, Example 43 and Comparative Examples 50 to 54 as shown in Table 15 and Table 16 by the below-mentioned method, the cosmetic materials were evaluated by the below-mentioned evaluation method and evaluation criteria. In the case of the water-in-oil foundations, the "inhibitory effect on secondary adhesion of the cosmetic material", the "flexibility of cosmetic film", the "strength of water repellency" and the "persistence of water repellency" were evaluated. Further, in the case of the oil-in-water moisturizing creams, the "flexibility of cosmetic film", the "strength of water repellency" and the "persistence of water repellency" were evaluated.

### [Method for Evaluating Inhibitory Effect on Secondary Adhesion of Cosmetic Material]

(I) A 3 cm × 3 cm region on the inside of a forearm was washed to remove any sebum. Three µL of macadamia nut oil was then applied to the prepared region as a substitute sebum for the removed sebum.
(II) Next, 18 µL of the cosmetic material being evaluated was applied on top of the prepared region and left to stand for three minutes.
(III) Tissue paper was placed on top to cover the applied region and pressed lightly from above, and the degree of transfer of a pigment to the tissue paper was evaluated.

### [Calculation of Secondary Adhesion Area]

Calculation of the secondary adhesion area was performed in the same manner as that described in the "Calculation of Secondary Adhesion Area" relating to the aforementioned liquid oily composition.

The captured photographs (actual images) of Example 42 and Comparative Example 50, as well as the images obtained by subjecting those photographs to binarization processing are shown in FIG. 2.

### [Evaluation Criteria of Inhibitory Effect on Secondary Adhesion of Cosmetic Material]

The inhibitory effect on secondary adhesion of a pigment was evaluated using the proportion of the detected area of the black portion calculated in the above-mentioned "Calculation of Secondary Adhesion Area".
A: The detected area was less than 15%.
B: The detected area was at least 15% but less than 20%.
C: The detected area was at least 20% but less than 25%.
D: The detected area was 25% or higher.

In the case of the grade D evaluated when the detected area was 30% or higher, no inhibitory effect was deemed to be exhibited.

### [Method for Evaluating Flexibility of Cosmetic Film]

(I) A 3 cm × 3 cm region on the inside of a forearm was washed to remove any sebum.
(II) Three µL of macadamia nut oil was applied to the prepared region as a substitute sebum for the removed sebum.
(III) Next, 18 µL of the cosmetic material being evaluated was applied on top of the macadamia nut oil and left to stand for 30 minutes.
(IV) The sensation at the applied region was then evaluated by ten monitors.

### [Evaluation Criteria of Flexibility of Cosmetic Film]

A: Eight to ten of the ten monitors sensed flexibility of the cosmetic film.
B: Five to seven of the ten monitors sensed flexibility of the cosmetic film.
C: Two to four of the ten monitors sensed flexibility of the cosmetic film.
D: One or less of the ten monitors sensed flexibility of the cosmetic film.

### [Method for Evaluating Strength of Water Repellency]

(I) Five µL of macadamia nut oil was applied to a 50 mm × 50 mm PMMA plate.
(II) Next, 20 µL of the cosmetic material being evaluated was applied thereon and left to stand for five minutes.
(III) Subsequently, 20 µL of ion-exchanged water was dripped onto the coating film applied to the plate, the plate was then inclined, and the angle at which the water droplet started to move was measured.

### [Evaluation Criteria of Strength of Water Repellency]

A: The water droplet started to move at a plate inclination angle of less than 40°.
B: The water droplet started to move at a plate inclination angle of at least 40° but less than 42°.
C: The water droplet started to move at a plate inclination angle of at least 42° but less than 43°.
D: The water droplet did not move even at a plate inclination angle of 43° or higher.

### [Method for Evaluating Persistence of Water Repellency]

(I) A PMMA plate prepared in the same manner as described above in the "Method for Evaluating Strength of Water Repellency " was immersed for ten minutes in ion-exchanged water held at 40°C, and then dried naturally.
(II) Subsequently, 20 µL of ion-exchanged water was dripped onto the coating film applied to the plate, the plate was then inclined, and the angle at which the water droplet started to move was measured.

### [Evaluation Criteria of Persistence of Water Repellency]

A: The water droplet started to move at a plate inclination angle of less than 34°.
B: The water droplet started to move at a plate inclination angle of at least 34° but less than 35°.
C: The water droplet started to move at a plate inclination angle of at least 35° but less than 36°.
D: The water droplet did not move even at a plate inclination angle of 36° or higher.

### [Water-in-Oil Foundation: Overall Evaluation of Inhibitory Effect on Secondary Adhesion, Flexibility of Cosmetic Film, Strength of Water Repellency and Persistence of water repellency]

Each of the grades of the cosmetic material obtained by evaluating the inhibitory effect on the secondary adhesion of a pigment, the flexibility of cosmetic film, the strength of water repellency and the persistence of water repellency was converted to a numerical score by assigning four points to the grade A, three points to the grade B, two points to the grade C, and one point to the grade **D,** and the sum of the numerical scores was used as an overall evaluation score.

### [Oil-in-Water Moisturizing Cream: Overall Evaluation of Flexibility of Cosmetic Film, Strength of Water Repellency and Persistence of water repellency]

Each of the grades of the cosmetic material obtained by evaluating the flexibility of cosmetic film, the strength of water repellency and the persistence of water repellency was converted to a numerical score by assigning four points to the grade A, three points to the grade **B,** two points to the grade C, and one point to the grade **D,** and the sum of the numerical scores was used as an overall evaluation score.

### (Water-in-Oil Foundation)

Each water-in-oil foundation (each cosmetic material according to the present invention) was produced by preparing the liquid oily composition of Example **1,** namely by mixing dimethicone 10 cs as the component (A), diisostearyl malate as the component (B), and ethylhexyl hydroxystearate as the component (C) such that the mass ratio (B)/(C) was 3.0 and the mass ratio {(B)+(C)}/(A) was 4.0, and then blending the liquid oily composition to obtain a water-in-oil foundation.

The constitutions and evaluation results of the water-in-oil foundations are shown in Table 15. The product "KF-6017" manufactured by Shin-Etsu Chemical Co., Ltd. was used as a polyether-modified silicone. The product "KF-7312J" manufactured by Shin-Etsu Chemical Co., Ltd. was used as trimethylsiloxysilicate used as a film-forming agent.

### [Method for Producing Water-in-Oil Foundation]

The water-in-oil foundation was produced by the following steps a to c.
a: Components 1 to 10 were dissolved and mixed uniformly under heat.
b: Components 11 to 13 were dissolved and mixed uniformly under heat.
c: The mixture obtained in the step b at 80°C was added to the mixture obtained in the step a at 80°C, and the resulting mixture was emulsified and then cooled.

**[Table 15]**

| Table 15 | Constitution (% by mass) of water-in-oil foundations, and evaluation results | | | | |
|---|---|---|---|---|---|
| Components (raw materials) | | Example 42 | Comparative Example 50 | Comparative Example 51 | Comparative Example 52 |
| 1 (Component (A)) | Dimethicone 10 cs | 8 | 0 | 8 | 0 |
| 2 (Component (B)) | Diisostearyl malate | 24 | 32 | 32 | 32 |
| 3 (Component (C)) | Ethylhexyl hydroxystearate | 8 | 8 | 0 | 8 |
| 4 | Polyether-modified silicone | 1 | 1 | 1 | 1 |
| 5 | Trimethoxysiloxysilicate | 0 | 0 | 0 | 5 |
| 6 | Talc | 2.3 | 2.3 | 2.3 | 2.3 |
| 7 | Titanium oxide | 7 | 7 | 7 | 7 |
| 8 | Red iron oxide | 0.5 | 0.5 | 0.5 | 0.5 |
| 9 | Yellow iron oxide | 0.1 | 0.1 | 0.1 | 0.1 |
| 10 | Black iron oxide | 0.1 | 0.1 | 0.1 | 0.1 |
| 11 | Methylparaben | 0.1 | 0.1 | 0.1 | 0.1 |
| 12 | Sodium chloride | 1 | 1 | 1 | 1 |
| 13 | Ion-exchanged water | 47.9 | 47.9 | 47.9 | 42.9 |
| Total | | 100 | 100 | 100 | 100 |
| Dimethicone compatibility value of component (B) | | 2. 94 | | | |
| Dimethicone compatibility value of component (C) | | -1. 18 | | | |
| (B)/(C) | | 3.00 | 4.00 | - | 4.00 |
| {(B)+(C)}/(A) | | 4.00 | - | 4.00 | - |
| Compatibility 1 | | S | S | S | S |
| Compatibility 2 | | IS | S | S | S |
| Viscosity of mixture of component (B) and component (C) (25°C, mPa·s) | | 550 | 725 | 3100 | 725 |
| Evaluation results | Inhibition on secondary adhesion | A | D | D | D |
| | Flexibility of cosmetic film | A | A | A | D |
| | Strength of water repellency | A | D | D | C |
| | Persistence of water repellency | A | D | D | C |
| | Overall evaluation | 16 | 7 | 7 | 6 |

As is evident from Table 15, it was confirmed that the cosmetic material of Example 42 had an excellent inhibitory effect on secondary adhesion of a pigment, favorable flexibility of the silicone film, and excellent water repellency with strength and persistence. The cosmetic material of Comparative Example 50, in which a liquid oily composition that did not contain the component (A) was used, and the cosmetic material of Comparative Example 51, in which a liquid oily composition that did not contain the component (C) was used, exhibited favorable flexibility of the cosmetic film, but the inhibitory effect on secondary adhesion of a pigment and the strength and persistence of water repellency thereof were poor. Although the cosmetic material of Comparative Example 52 was formed by adding trimethylsiloxysilicate to the formulation of Comparative Example 50 as a film-forming agent, the inhibitory effect on secondary adhesion and the strength and persistence of water repellency were poor, and the flexibility of the cosmetic film was also inferior.

Based on these results, it was evident that in those cases where the compatibility 1 was evaluated as "S" and the compatibility 2 was evaluated as "IS", the separated dimethicone (the component (A)) formed a silicone film, thereby exhibiting the effects of the present invention.

### (Oil-in-Water Moisturizing Cream)

Each oil-in-water moisturizing cream was produced by preparing the liquid oily composition of Example **1,** namely by mixing dimethicone 10 cs as the component (A), diisostearyl malate as the component (B), and ethylhexyl hydroxystearate as the component (C) such that the mass ratio (B)/(C) was 3.0 and the mass ratio {(B)+(C)}/(A) was 4.0, and then blending the liquid oily composition to obtain each oil-in-water moisturizing cream.

The constitutions and evaluation results of the oil-in-water moisturizing creams are shown in Table 16. The product "KALCOL 6870" manufactured by Kao Corporation was used as cetanol, the product "SALACOS PGMSV" manufactured by The Nisshin OilliO Group, Ltd., was used as polyglyceryl-10 stearate, and the product "SALACOS PGDSV" manufactured by The Nisshin OilliO Group, Ltd., was used as polyglyceryl-10 distearate.

### [Method for Producing Oil-in-Water Moisturizing Cream]

The oil-in-water moisturizing creams were each produced by the following steps a to c.
a: Components 1 to 4 were dissolved and mixed uniformly under heat.
b: Components 5 to 8 were dissolved and mixed uniformly under heat.
c: The mixture obtained in the step a at 80°C was added to the mixture obtained in the step b at 80°C, and the resulting mixture was emulsified and then cooled.

**[Table 16]**

| Table 16 | Constitution (% by mass) of oil-in-water moisturizing creams, and evaluation results | | | |
|---|---|---|---|---|
| Components (raw materials) | | Example 43 | Comparative Example 53 | Comparative Example 54 |
| 1 (Component (A)) | Dimethicone 10 cs | 4 | 2 | 0 |
| 2 (Component (B)) | Diisostearyl malate | 12 | 18 | 15 |
| 3 (Component (C)) | Ethylhexyl hydroxystearate | 4 | 0 | 5 |
| 4 | Cetanol | 3 | 3 | 3 |
| 5 | Polyglyceryl-10 stearate | 1.5 | 1.5 | 1.5 |
| 6 | Polyglyceryl-10 distearate | 1.5 | 1.5 | 1.5 |
| 7 | Methylparaben | 0.2 | 0.2 | 0.2 |
| 8 | Ion-exchanged water | 73.8 | 73.8 | 73.8 |
| Total | | 100 | 100 | 100 |
| Dimethicone compatibility value of component (B) | | 2.94 | | |
| Dimethicone compatibility value of component (C) | | -1.18 | | |
| (B)/(C) | | 3.00 | - | 3.00 |
| {(B)+(C)}/(A) | | 4.00 | 9.00 | - |
| Compatibility 1 | | S | S | S |
| Compatibility 2 | | IS | S | S |
| Viscosity of mixture of component (B) and component (C) (25°C, mPa·s) | | 550 | 3100 | 550 |
| Evaluation results | Flexibility of cosmetic film | A | A | A |
| | Strength of water repellency | A | D | D |
| | Persistence of water repellency | A | D | D |
| | Overall evaluation | 12 | 6 | 6 |

As is evident from Table 16, it was confirmed that the cosmetic material of Example 43 exhibited flexibility and excellent water repellency with strength and persistence when applied to form a coating film. In contrast, the cosmetic films formed in Comparative Examples 53 and 54 had excellent flexibility, but the strength and persistence of the water repellency thereof were poor.

Based on these results, it was evident that in those cases where the compatibility 1 was evaluated as "S" and the compatibility 2 was evaluated as "IS", the separated dimethicone (the component (A)) formed a silicone film, thereby exhibiting the effects of the present invention.

### INDUSTRIAL APPLICABILITY

The present invention makes it possible to provide a liquid oily composition which imparts an inhibitory effect on secondary adhesion and water repellency with strength and persistence to a cosmetic material, as well as a cosmetic material containing the liquid oily composition.

## Claims

1. A liquid oily composition comprising:
a component (A): a dimethicone having a kinematic viscosity at 25°C within a range from 4 to 12 mm²/s,
a component (B): an ester oil that is liquid at 25°C and has a dimethicone compatibility value, determined using a formula (I) shown below, of at least 1.75, and
a component (C): an ester oil that is liquid at 25°C and has a dimethicone compatibility value, determined using the formula (I) shown below, of less than 1.75, wherein
the liquid oily composition is transparent and uniform at 25°C, and
the component (A) is separated from a mixture prepared by adding 8 parts by mass of macadamia nut oil to 100 parts by mass of the liquid oily composition, [Numerical Formula 1] the dimethicone compatibility value of the ester oil = 10,000 × (a carbon branching value + an oxygen value) / (a molecular weight of the ester oil)2 (1)
wherein the carbon branching value = (a number of carbon branches within a molecular structure of the ester oil × 12), and the oxygen value = (-3 × a number of hydroxyl groups within the molecular structure of the ester oil × 16) + (0.5 × a number of ester groups within the molecular structure of the ester oil × 2 × 16) + (-0.1 × a number of ether groups in the ester oil × 16).

2. The liquid oily composition according to Claim 1, wherein
a mass ratio ((B)/(C)) of the component (C) to the component (B) is within a range from 0.32 to 18.00, and
a mass ratio ({(B)+(C)}/(A)) of the component (A) to a total of the component (B) and the component (C) is within a range from 1.50 to 12.33.

3. The liquid oily composition according to Claim 1, wherein
the component (B) is one type or at least two types of ester oil selected from the group consisting of isononyl isononanoate, isotridecyl isononanoate, neopentyl glycol diethylhexanoate, glyceryl triethylhexanoate, glyceryl triisostearate (having a 2-(1,3,3-trimethylbutyl)-5,7,7-trimethyloctyl structure in the aliphatic chain), diisostearyl malate (having a 2-(1,3,3-trimethylbutyl)-5,7,7-trimethyloctyl structure in the aliphatic chain), pentaerythrityl tetraethylhexanoate, neopentyl glycol dicaprate, propanediol di(caprylate/caprate), polyglyceryl-2 triisostearate (having a 2-(1,3,3-trimethylbutyl)-5,7,7-trimethyloctyl structure in the aliphatic chain), cetyl ethylhexanoate, ethylhexyl palmitate, glyceryl tri(caprylate/caprate), and dipentaerythrityl pentaisostearate (having a 2-(1,3,3-trimethylbutyl)-5,7,7-trimethyloctyl structure in the aliphatic chain).

4. The liquid oily composition according to Claim 1, wherein
the component (C) is one type or at least two types of ester oil selected from the group consisting of propanediol diisostearate, trimethylolpropane triisostearate, pentaerythrityl tetraisostearate, polyglyceryl-2 tetraisostearate, polyglyceryl-2 triisostearate, and ethylhexyl hydroxystearate.

5. The liquid oily composition according to Claim 1, wherein
a viscosity at 25°C of a mixture of the component (B) and the component (C) is within a range from 30 to 1,200 mPa·s.

6. A cosmetic material comprising a liquid oily composition of any one of Claims 1 to 5.

7. The cosmetic material according to Claim 6, further comprising a component (D): a powder.

8. The cosmetic material according to Claim 6, further comprising a component (E): water, and a component (F): a surfactant, wherein
the cosmetic material is an oil-in-water emulsion cosmetic material or a water-in-oil emulsion cosmetic material.

9. A method for producing a cosmetic material, comprising blending a liquid oily composition of any one of Claims 1 to 5 as a raw material.
